# EUROPEAN PATENT APPLICATION

(11) **EP 3 977 910 A1**
(43) Date of publication of application: **06.04.2022**
(21) Application number: 20815275.1
(22) Date of filing: 26.05.2020
(51) Int. Cl.: A61B 1/00, A61B 1/045, A61B 1/313, A61B 3/10

(54) **LESION DETECTION METHOD**

(30) Priority: 27.05.2019 JP 2019098860
(71) Applicant: Mie University, Tsu-shi, Mie 514-8507 (JP)
(72) Inventor: MIZOGUCHI, Akira, Tsu-shi Mie 514-8507 (JP); TANAKA Koji, Tsu-shi Mie 514-8507 (JP); KIMURA Kazushi, Tsu-shi Mie 514-8507 (JP); NOSAKA Tetsuya, Tsu-shi Mie 514-8507 (JP); TANAKA Kyosuke, Tsu-shi Mie 514-8507 (JP); WANG Shujie, Tsu-shi Mie 514-8507 (JP); KAITO Aika, Tsu-shi Mie 514-8507 (JP); TOIYAMA Yuji, Tsu-shi Mie 514-8507 (JP); GOTO Hidemasa, Tsu-shi Mie 514-8507 (JP); SUGIMOTO Masahiko, Tsu-shi Mie 514-8507 (JP); NISHIMURA Yuuhei, Tsu-shi Mie 514-8507 (JP); GABAZZA Esteban, Tsu-shi Mie 514-8507 (JP)
(74) Representative: Lederer & Keller Patentanwälte Partnerschaft mbB
(86) International application number: PCT/JP2020/020721
(87) International publication number: WO 2020/241633

(57) **Abstract**

Provided is a method for detecting a lesion characterized by administering to an organ a cell staining agent that enables observation of biological tissue by laser irradiation, and then irradiating the organ with multiphoton laser or confocal laser to image the inside of lesion in the organ, and determining the interface between normal site and lesion site.

## Description

### TECHCAL FIELD

The present invention relates to a vital staining test method and a tissue visualization method to distinguish the lesion site and normal site in an organ using laser irradiation after vital staining of organ tissue with edible dyes, use of a cell staining agent for visualization of lesion in an organ, and a composition comprising a cell staining agent for the examination of lesion in an organ.

### BACKGROUND ART

Cancer is currently the leading cause of death in Japan, with one in two people suffering from cancer and one in four dying from cancer. Moreover, the number of deaths from cancer is still increasing, and reducing the number of deaths from cancer is a public desire. Although the basic strategy to reduce the deaths caused by cancer is considered to be early detection of cancer, there is a limit in current test methods using conventional endoscopy, since it is difficult to detect cancer with a diameter of less than about 10∼20 mm. Therefore, most cancer patients are currently treated by surgical resection of cancer and there is an urgent need to develop a technology for supporting rapid decisions before and during the surgeries.

The goal of surgical resection of cancer is to simultaneously achieve the extremely difficult goals of completely removing cancer cells as completely as possible and maximizing preservation of organ function after removal of lesion. Great improvements in surgical technics and efforts have been made to achieve this very difficult goal. One of the key points to improve a result of surgical cancer treatment has been rapid pathological diagnosis. It would be of great help if a surgeon can accurately ascertain the extent to which cancer cells have invaded or metastasized in an organ that has developed cancer and its surrounding organs, as well as lymph nodes and blood vessels at pathological diagnosis level before and during surgical operations.

A method has been reported (Patent Literature 1) for distinguishing cancer cells from normal cells with devices such as a laser endomicroscope from luminal surface after staining digestive tract from luminal surface *in vivo* with edible dyes, such as curcumin, sulfuretin, Red #3, and Red #106, etc. Meanwhile, there are few effective methods for distinguishing cancer cells from normal cells from serosal surface or lumen of digestive tract or the like before or during surgical operations. Accordingly, there is a strong demand for developing such methods.

### PRIOR ART LITERATURE

### Patent Literature

1. WO2014/157703

### SUMMARY OF THE INVENTION

### Problems to be solved by the invention

With the introduction of a robot for endoscopic surgery in thoracic cavity or abdominal cavity, less invasive surgical operations are performed without laparotomy, and the burden on patients is greatly reduced. On the other hand, in case of cancer, in order to avoid recurrence due to micro invasion, even if a robot for endoscopic surgery in thoracic cavity or abdominal cavity is used, a resection site has to be extensive. For reducing the burden on patients, it is necessary to reduce the resection extent. Since surgical operations are basically performed from the serosal side of an organ, before and during surgical operations, if it is possible to predict the extent of invasion of cancer cells from the serosal side before resection of the affected parts, the resection extent can be reduced. The burden on cancer patients can be significantly reduced combined with advantages of a robot for endoscopic surgery in thoracic cavity or abdominal cavity. For this purpose, a technique for clarifying cancer tissue stump from serosal side is required. In addition, for example, when taking a capsule endoscope orally, it is necessary to observe cancer tissue from lumen of digestive tract.

### Means for solving the problems

The present inventors have been developing a rapid intraoperative pathological diagnosis system using cancer cell-specific vital staining and a laser microscope and have found out that cancer cells are stained more densely than normal cells when observing vital-stained cells with a laser microscope after coating certain edible dyes such as curcumin to mucosal surface of digestive tract. The staining allowed rapid detection of cancer cells and further enabled clear visualization of cell morphology including nucleus morphology of cells. As a result, cellular atypia and structural atypia can be reliably distinguished, and a method for detecting and treating micro-cancers was successfully developed. In this method, a sterile solution of about 1 mg/mL of an edible dye approved for human ingestion such as curcumin and Red #3 is coated to *in vivo* luminal surface of digestive tract, lymph nodes or *ex vivo* resection samples, then allowed to stand for about 1∼5 minutes, and imaged within a few seconds with a laser microscope. Accordingly, this technique can greatly contribute to rapid intraoperative pathological diagnosis.

Since robot techniques for endoscopic surgery in thoracic cavity or abdominal cavity are basically performed from serosal side of an organ, if it is possible to predict the extent of invasion of cancer cells from the serosal side before a surgery and determine whether or not cancer cells still remain after the surgery, radical resection of cancer will be possible and at the same time, the resection extent will be small. Therefore, the burden on patients will be greatly reduced.

The present invention provides a method for detecting lesion by administering to an organ a cell staining agent that enables observation of biological tissue by laser irradiation, and then irradiating the organ with multiphoton laser or confocal laser to obtain the histological images of optical sections inside of a lesion in the organ, before conforming the interface between the normal site and the lesion site.

That is, the present invention is as follows.
[1] A method for detecting lesion characterized by administering to an organ a cell staining agent that enables observation of biological tissue with laser irradiation, then irradiating the organ with multiphoton laser or confocal laser, obtaining the histological images of optical sections inside of a lesion in the organ, and conforming the interface between the normal site and the lesion site.
[2] The method according to [1], wherein the inside of a lesion is a micro-lesion with a diameter of about 5∼500 µm.
[3] The method according to [1] or [2], wherein the organ is irradiated from its serosal side or lumen with multiphoton laser or confocal laser.
[4] The method according to any one of [1] ∼ [3], wherein the cell staining agent is one or more staining agents selected from the group consisting of sulfuretin, curcumin, Red #3 (erythrosine), and Red #106.
[5] The method according to any one of [1] ∼ [4], wherein the administration of a cell staining agent is performed by coating, dropping or spraying from the serosal side of an organ.
[6] The method according to any one of [1] ∼ [4], wherein the administration of a cell staining agent is performed by coating, dropping or spraying from the lumen of an organ.
[7] The method according to any one of [1] ∼ [4], wherein the administration of a cell staining agent is oral administration, intravenous administration, intraperitoneal administration, subcutaneous injection, intramuscular injection, intraorgan injection, intrathoracic administration, or subarachnoid administration.
[8] The method according to any one of [1] ∼ [7], wherein the laser irradiation is performed by using a multiphoton laser microscopic endoscope, a confocal laser microscopic endoscope, or a laser fluorescent microscopic endoscope.
[9] The method for detecting cancer cells, characterized by using the method according to [8] to visualize cancer cells.
[10] The method according to [9] for determining invasion of cancer to regional lymph node tissues when cancer cells exist in an organ suspected of presence of cancer, which comprises administering to lymph node tissues a cell staining reagent that enables observation of biological tissue with laser irradiation by dropping from the surface covering the lymph node tissues or injecting into the lymph nodes, and then irradiating the lymph node tissues with multiphoton laser or confocal laser.
[11] The method according to [9], characterized by staining tissue in an organ suspected of presence of cancer cells with curcumin or sulfuretin, then laser irradiating the organ tissue from its serosal side or lumen using a multiphoton laser microscopic endoscope, a confocal laser microscopic endoscope, or a laser fluorescent microscopic endoscope, and identifying normal or cancer cells based on visualized images obtained on cytoplasmic and nuclear morphology of the cells present in the organ tissue.
[12] The method according to [11], wherein the organ is a respiratory, digestive, or genitourinary organ.
[13] The method according to [9], characterized by staining tissue in an organ suspected of presence of cancer with curcumin, then laser irradiating the organ tissue from its serosal side or a lumen using a multiphoton laser microscopic endoscope, a confocal laser microscopic endoscope, or a laser fluorescence microscopic endoscope, comparing the crypt structures of cancer tissue and normal tissue present in the visualized organ tissue, and determining the lesion site as cancer according to the observation of disappearance of the regular crypt structure found in normal tissue and populations of disordered cell proliferation of cancer cells that do not have the crypt structures.
[14] The method according to [9], characterized by staining tissue in an organ suspected of presence of cancer with Red #106, then laser irradiating the organ tissue from its serosal side or lumen using a multiphoton laser microscopic endoscope, a confocal laser microscopic endoscope, or a laser fluorescent microscopic endoscope, comparing the patterns of the capillaries around cancer cells and normal cells in the visualized organ tissue, and detecting the cancer cells according to the observation of disappearance and/or deformation of the capillaries in the regular crypt structure found in normal tissue.
[15] The method according to [9], characterized by vital staining epithelial cells and cancer cells with curcumin, or connective tissue and capillaries with Red #106 in an organ suspected of presence of cancer, then laser irradiating the organ tissue from its serosal side or lumen using a multiphoton laser microscopic endoscope, a confocal laser microscopic endoscope, or a laser fluorescent microscopic endoscope, conforming the boundary between the cancer cells and the connective tissue existing in the visualized organ tissue, and determining the infiltration of the cancer cells.
[16] The method according to [9], which comprises staining an organ tissue with curcumin or sulfuretin in an organ suspected of presence of cancer, then laser irradiating the organ tissue from its serosal side or lumen using a multiphoton laser microscopic endoscope, a confocal laser microscopic endoscope or a laser fluorescent microscopic endoscope, and visualizing Meissner's plexus or Auerbach's plexus present in the organ tissue.
[17] The method according to [16], characterized in that when a primary lesion of cancer is in mucosal epithelium, if the cancer cells have invaded or reached Meissner's plexus, the cancer is determined as an advanced cancer.
[18] The method according to [16], characterized in that when a primary lesion of cancer is in mucosal epithelium, if the cancer cells have invaded or reached the Meissner's plexus and smooth muscle layer, the cancer is determined as an advanced cancer.
[19] The method according to [16], characterized in that when a primary lesion of cancer is in mucosal epithelium, if the cancer cells have not invaded or reached Meissner's plexus, the cancer is determined as an early cancer.
[20] The method according to [16], characterized by comprehensively observing the interface between cancer tissue and normal tissue surrounding the ultra-early cancer tissue, and determining whether or not the cancer has infiltrated and metastasized according to the image of the interface.
[21] The method according to any one of [9] ∼ [20], which further comprises notifying the detection of cancer cells by sound or light.
[22] The method for treating cancer patients by removing cancer cells one by one from serosal side or lumen, characterized by using any one of the methods according to [9] ∼ [21].
[23] The method for confirming cancer cells remaining *in vivo* from serosal side or lumen after a surgery, and removing the cancer cells one by one, characterized by using any one of the methods according to [9] ∼ [21].
[24] A method for diagnosing the tissue-type of lung cancer, characterized by using any one of the methods according to [9] ∼ [21].
[25] A method for treating patients with lung cancer, characterized by using the method according to [24].
[26] The method according to [25], wherein lung cancer cells are destroyed by laser transpiration with laser beam.
[27] A method for visualizing brain tissue, characterized by using the method according to [8] to fluorescently label neurons in the brain tissue with a staining agent and determine the morphology of the neurons.
[28] A method according to [27], wherein the brain tissue is cerebral cortex, hippocampus, amygdala, hypothalamus, or cerebellum.
[29] A method for detecting a brain disease or brain symptom using visualized images obtained by the method according to [27] or [28].
[30] A method according to [29], wherein the brain diseases or brain symptom comprise Alzheimer's disease, cerebral infarction, cerebral hemorrhage, subarachnoid hemorrhage, multiple sclerosis, and spinocerebellar degeneration.
[31] A method for visualizing ocular tissue, characterized by using the method according to [8] to fluorescently label neurons in the ocular tissue with a staining agent and determine the morphology of the neurons.
[32] A method according to [31], wherein the ocular tissue is retina.
[33] A method for detecting an ophthalmic disease or ophthalmic symptom, using the visualized images obtained by the method according to [31] or [32].
[34] A method according to [33], wherein the ophthalmic disease or ophthalmic symptom comprise macular degeneration, retinal degeneration, diabetic retinopathy, retinoblastoma, proliferative vitreoretinopathy, glaucoma, retinal detachment, and retinal edema.
[35] A method for detecting whether or not cancer cells exist in lymph nodes during a laparoscopic surgery before lymph node resection, characterized by using the method according to [10].
[36] A cancer immunotherapy characterized by visualizing the cancer cells that have metastasized to lymph nodes by the method according to [9], destroying only cancer cells one by one by laser transpiration, letting lymphocytes recognize the cancer-related antigens of the destroyed cancer cells, and letting activated lymphocytes attack cancer cells in the cancer primary lesion.
[37] A method for diagnosing a disease that causes abnormality in location, number, shape, size, and arrangement of cells by visualizing cell structure of neuronal cells in digestive tract, brain and retina, sensory cells of taste and smell, endocrine cells, lymph nodes, skeletal muscle, lungs, pancreas, or liver by oral or intraperitoneal administration of curcumin, and imaging the visualized cell structure with a laser microscopic endoscope or a fluorescent microscope.
[38] A method for destroying and removing abnormal cells one by one by laser irradiation in a disease diagnosed by using the method according to [37].
[39] A cell removal method wherein when transplanted iPS cells are transformed into undifferentiated cells or cancer cells, the altered cells are visualized by the method according to [9], and only the altered cells are destroyed by laser ablation .
[40] A method for diagnosing the cause of tonsillitis, characterized by using the methods according to [8].
[41] A method according to [40], characterized by staining out polynuclear leukocytes, lymphocytes, and eosinophils by multiple staining with three or more kinds of edible dyes.
[42] A method according to [41] regarding cell types of leukocytes infiltrating tonsils, which comprises steps of detecting as bacterial infectious tonsillitis when a large number of neutrophils infiltrate, detecting as allergic tonsillitis when a large number of eosinophils infiltrate, and detecting as viral infectious tonsillitis when a large number of lymphocytes infiltrate.
[43] A method for visualizing skeletal muscle, characterized by using the method according to [8].
[44] A method of analyzing the morphology of skeletal muscle by using the visualization method according to [43] to determine the cause for muscle weakness due to aging.
[45] A method for analyzing the morphology of skeletal muscle by using the visualization method according to [43] to diagnose the lesions of sarcopenia and/or myasthenia gravis.
[46] A method for detecting a lesion, comprising the steps of administering a staining agent to an organ for a sufficient period and at a sufficient amount that enable the visualization of the lesion in the organ, irradiating the organ with multiphoton laser or confocal laser, and visualizing the lesion of the organ.
[47] Use of a cell staining agent for a lesion in an organ, characterized by administering the cell staining agent to the organ, then irradiating the organ with multiphoton laser or confocal laser, imaging the inside of lesion in the organ, and determining the interface between normal site and lesion site.
[48] A composition containing a cell staining agent for detecting a lesion in an organ, characterized in that after the composition is administered to the organ, the organ is irradiated with multiphoton laser or confocal laser, the inside of lesion in the organ is imaged, and the interface between normal site and lesion site is determined.
[49] Use of a multiphoton laser microscopic endoscope, a confocal laser microscopic endoscope, or a laser fluorescent microscopic endoscope for a lesion in an organ, characterized by administering a cell staining agent to the organ, then irradiating the organ with multiphoton laser or confocal laser, imaging the inside of lesion in the organ, and determining the interface between normal site and lesion site.

### EFFECTS OF THE INVENTION

According to the present invention, it becomes possible to detect a micro lesion with a diameter of about 5∼500 µm by obtaining histological images of optical sections inside of a lesion in an organ. That is, it becomes possible to detect a micro lesion related to capillaries with a diameter of about 5 µm and an ultra-early cancer with a diameter of about 500 µm, such as epithelial cancer.

According to the present invention, it is possible to visualize the morphology of cells at the depth of 0.05∼1.0 mm from surface of an organ. For example, in cases of ultra-early cancer in large intestine, internal observation at a depth of about 500 µm from its serosal surface is possible, and internal observation at a depth of about 500 µm from its mucosal surface is also possible. Therefore, as long as the whole layer of tissue has a thickness of about 1000 µm, the whole layers of the tissue can be observed from both sides (i.e. the serosal surface and mucosal surface). Accordingly, the whole image of the ultra-early cancer in large intestine can be observed.

By using the detection method of the present invention, it becomes possible to perform early diagnosis and treatment of diseases. According to the present invention, since the dyes for staining tissue penetrate into the inside of the tissue, the whole ultra-early cancer having a diameter of about 0.2 mm can be completely imaged. Therefore, it becomes possible to diagnose and treat ultra-early cancer according to the present invention. In addition, in case of ultra-early cancer, it is possible to comprehensively observe the interface between cancer tissue and normal tissue based on the whole image of the visualized ultra-early cancer, and detect whether or not the cancer has infiltrated and transferred based on the observation result of the interface.

According to the present invention, since the dyes for staining a tissue penetrate into the inside of tissue, the whole lesion with a diameter of about 1 mm can be completely imaged even in case of a lesion in retina or brain. Therefore, it becomes possible to accurately determine the range of a lesion in retina or brain according to the present invention.

It becomes possible to simultaneously identify multiple cells and tissue structures by multiple staining with a plurality of edible dyes according to the present invention. For example, it becomes possible to simultaneously identify three or more types of cells and tissue structures, such as cancer cells, lymphocytes, and blood vessels by multiple staining with three or more kinds of edible dyes according to the present invention.

According to the method of the present invention, a solution of sulfuretin, curcumin, Red #3, or Red #106, which are edible dyes approved for human ingestion, is sprayed from luminal or serosal side of a tissue and allowed to stand for about 1∼5 minutes, or a solution of curcumin is orally administered, and cell morphology can be imaged and identified from the serosal side with a multiphoton laser microscope or a confocal laser microscope. Curcumin and Red #3 can stain cells that overexpress STAT3 and RAS, which are cancer-related gene products respectively. When the dye is sprayed according to the method, it takes less than 5 minutes to achieve images. A surgeon can choose a plurality of tissue sites of interest continuously. That is, a method for identifying tissue and cells necessary for a surgeon to make an immediate pathological diagnosis during surgical operations is provided by the present invention. Because the images from a multiphoton laser microscope or a confocal laser microscope obtained by the method are very clear, and nuclear morphology of individual cells is clearly visualized, cellular atypia and structural atypia of cancer can be detected reliably.

### BRIEF DESCRIPTION OF FIGURES

FIG. 1 is a schematic diagram showing stepwise mutation and activation of an oncogenic gene of a living cell group on inner wall of digestive tract, and process of cancer development, invasion and metastasis.
FIG. 2 is a diagram showing an example of a proliferation curve of human cancer cells.
FIG. 3 shows photographs taken with a multiphoton laser microscope from lumen side of an isolated large intestine of a mouse (normal mucosal tissue and cancer tumor site) stained with curcumin from the lumen side, wherein (A) is a photograph of normal mucosa of large intestine, and (B) is a photograph of a tumor site of colon cancer.
FIG. 4 shows photographs taken with a multiphoton laser microscope from lumen side of an isolated large intestine of a mouse (normal mucosal tissue) vital-stained with curcumin and Red #106 from lumen side.
FIG. 5a is a diagram illustrating tissue structure of a normal large intestine, which shows epithelial and glandular layers (1), muscularis mucosae (2), submucosal layer (3), muscle layer (4), and serosa (5) in the order from the surface facing the lumen through which food passes towards deep parts. Typical histological images taken by multiphoton microscopy at the focal planes A, B, C, and P are shown.
FIG. 5b shows serial photographs taken with a multiphoton laser microscope from serosal side while changing focal planes from an isolated large intestine of a mouse (normal mucosal tissue) stained with curcumin from its lumen side. The numbers above the photographs indicate focal planes from the serosal side.
FIG. 5c is an image, at low magnification, constructed by serial z-stack images taken with a multiphoton laser microscope of Auerbach's plexus in positively stained muscle layer by vital staining an isolated large intestine of a mouse (normal mucosal tissue) with curcumin from serosa side. This photograph is taken by focusing on muscle layer (4) (see FIG. 5a).
FIG. 5d shows, at high magnification, an example of visualization with a multiphoton laser microscope of Auerbach's plexus in vital-stained muscle layer with curcumin from serosa side in an isolated large intestine of a mouse (normal mucosal tissue), and that perikaryon can be identified.
FIG. 5e shows an image taken with a multiphoton laser microscope of Auerbach's plexus in muscle layer in an isolated large intestine of a mouse (normal mucosal tissue) visualized by vital staining with curcumin from serosa side.
FIG. 5f is a diagram visualizing thick blood vessels and smooth muscle of an isolated large intestine of a mouse (normal mucosal tissue) by vital staining with Red #106 and imaging from its serosal side with a multiphoton laser microscope. This photograph was taken by focusing on muscle layer (4) (see FIG. 5a), and it shows that the smooth muscle and blood vessel wall are stained by Red #106.
FIG. 5g is a diagram visualizing glandular structure and crypt structure of an isolated large intestine of a mouse (normal mucosal tissue) by vital staining with Red #106 and imaging from its serosal side with a multiphoton laser microscope.

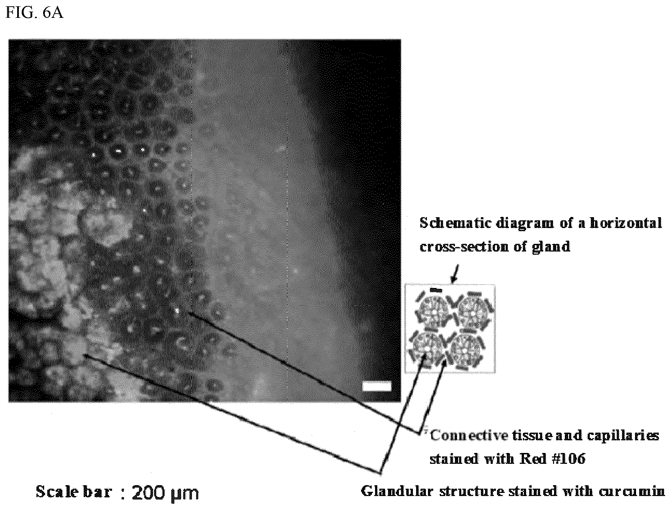

FIG. 6A is a photograph taken with a confocal laser microscope from serosal side by focusing on epithelial and glandular layers (1) (see FIG. 5a) after incising the abdomen of a mouse and double staining the tissue of large intestine with curcumin and Red #106 from serosal side of the large intestine (normal tissue). As indicated by the figure, curcumin positively stains gland cells, so that glandular structure is visualized. Red #106 positively stains connective tissue and capillaries as circumferential structures, so that crypt structure can be identified.

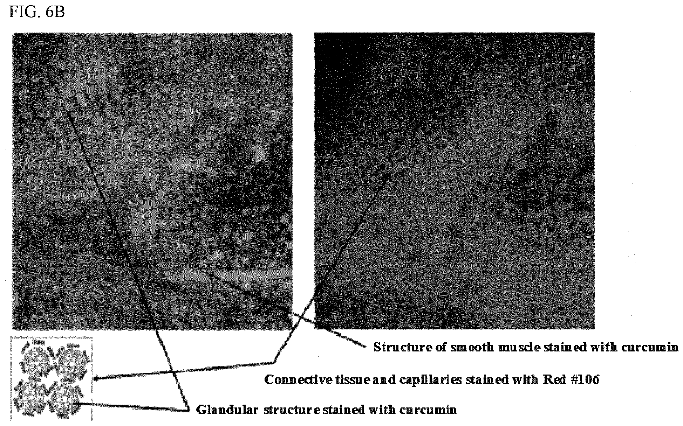

FIG 6B are photographs taken with a confocal laser microscope from serosal side by focusing on epithelial and glandular layers (1) and muscularis mucosae (2) (see FIG. 5a) after incising the abdomen of a mouse and double staining the tissue of large intestine with curcumin and Red #106 from serosal side of the large intestine (normal tissue). As indicated by the figure, glands are stained by curcumin. Connective tissue and capillaries are stained by Red #106. Furthermore, smooth muscle (muscularis mucosae) is stained by curcumin.

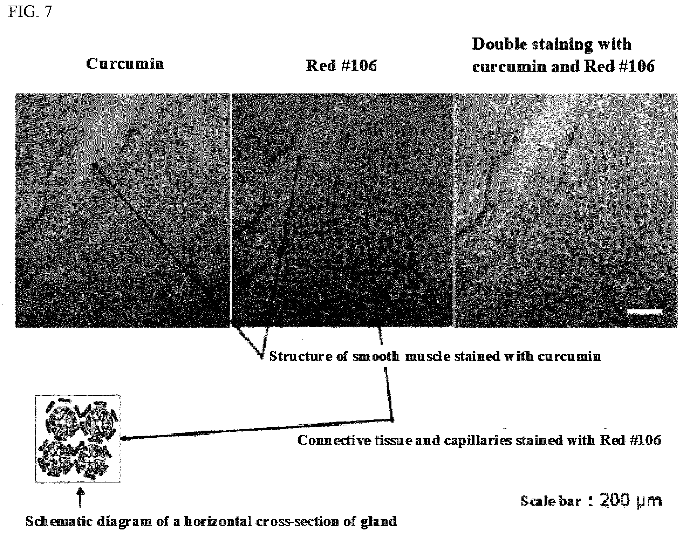

FIG. 7 are photographs taken with a confocal laser microscope from serosal side by focusing on epithelial and glandular layers (1) and muscularis mucosae (2) (see FIG. 5a) after incising the abdomen of a mouse and staining the tissue of large intestine with curcumin and Red #106 from luminal side of the large intestine (normal tissue). As indicated by the figure, also in the case of staining from luminal side, smooth muscle (muscularis mucosae) is stained by curcumin, while connective tissue and capillaries are stained by Red #106.
FIG. 8 is a schematic diagram showing staging according to local invasion and metastasis of cancer and treatment strategy.
FIG. 9 illustrates cellular architecture and local invasion of an early caner and an advanced cancer.
FIG. 10A is a photograph taken with a multiphoton laser microscopy from serosal side after incising the abdomen of a colon cancer mouse, and staining the tissue of large intestine from the serosal side of the large intestine (cancer tumor site) with curcumin. The two arrows in the figure indicate smooth muscle layer and cancer cells, respectively.
FIG. 10B is a photograph taken with a confocal laser microscopy from serosal side after incising the abdomen of a colon cancer mouse, and staining the tissue of large intestine from the serosal side of the large intestine (cancer tumor site) with curcumin. The three arrows in the figure indicate blood vessels (center), smooth muscle layers (right), and cancer cells (left), respectively.
FIG. 11A is a photograph taken with a multiphoton laser microscope from luminal side after incising the abdomen of a colon cancer mouse, and staining the tissue of large intestine (cancer tumor site) with curcumin from the luminal side.
FIG. 11B is a photograph taken with a multiphoton laser microscope from luminal side after incising the abdomen of a colon cancer-bearing mouse, and staining the tissue of large intestine (cancer tumor site) with curcumin from the luminal side showing the cellular atypia and structural atypia of the colon cancer.
FIG. 12 is a photograph taken with a multiphoton laser microscope from serosal side after incising of the abdomen of a colon cancer mouse, and staining the tissue of large intestine (cancer tumor) with Red #106 from the serosal side.
FIG. 13 shows photographs taken with a multiphoton laser microscope inserted into a mouse chest after incising the mouse chest, and staining normal lung tissue with curcumin (A) or Red #106 (B) from the surface of pleural side. As indicated by the photographs, the structure of alveolar can be clearly observed.
FIG. 14 is a diagram showing a cancer testing device (201) according to the present embodiment.
FIG. 15 illustrates a cancer test.
FIG. 16 is a diagram showing, at high magnification, an example of visualization with a multiphoton laser microscope of Auerbach's plexus in muscle layer after vital staining with curcumin from serosa side in a mouse.
FIG. 17 is a diagram showing, at high magnification, an example of visualization with a multiphoton laser microscopy of autonomic plexus (Meissner's plexus) in the muscle layer after vital staining with curcumin from serosa side in a mouse, based on which perikaryon can be identified.
FIG. 18 is a diagram schematically showing the process of cancer cell invasion and stage classification of cancer and importance of cancer invasion beyond the Meissner's plexus to distinguish early cancers from advanced ones.
FIG. 19 shows, at high magnification, examples of visualization with a multiphoton laser microscope of exocrine cells and islets of Langerhans of pancreas after vital staining by intraperitoneal administration of curcumin to a mouse. Furthermore, it shows hematoxylin-eosin (HE) staining images of pancreatic tissue.
FIG. 20 is a diagram showing, at high magnification, an example of visualization with a multiphoton laser microscope of a taste bud, which is a taste sensory device, after vital staining by coating curcumin onto tongue mucosa of a mouse.
FIG. 21 is a diagram showing, at high magnification, examples of visualization with a multiphoton laser microscope of a taste bud, which is a taste sensory device, after vital staining by coating curcumin onto tongue mucosa of a mouse.
FIG. 22 is a diagram showing, at high magnification, examples of visualization with a multiphoton laser microscope of sensory neuronal cells of a taste bud, which is a taste sensory device, after vital staining by coating curcumin onto tongue mucosa of a mouse.
FIG. 23 is a diagram showing, at high magnification, examples of visualization with a multiphoton laser microscope of a retinal neuronal cell group after vital staining by intraperitoneal administration of curcumin to a mouse.
FIG. 24 is a diagram showing, at high magnification, an example of visualization with a multiphoton laser microscope of a retinal neuronal cell group after vital staining by intravitreal injection of curcumin to a mouse.
FIG. 25 is a diagram showing, at high magnification, examples of visualization with a laser multiphoton microscope of olfactory nerve fibers after vital staining by intraperitoneal administration of curcumin to a mouse. As indicated by the figure, curcumin can stain myelin sheath.
FIG. 26 is a diagram showing, at high magnification, examples of visualization with a multiphoton laser microscope of olfactory receptor neurons, which are odor sensory cells, after vital staining by coating curcumin onto nasal mucosa of a mouse.
FIG. 27 is a diagram showing, at high magnification, examples of visualization with a multiphoton laser microscope of thyroid after vital staining by intraperitoneal administration of curcumin to a mouse. Furthermore, the figure shows a hematoxylin eosin (HE) staining image of thyroid tissue.
FIG. 28 is a diagram showing, at high magnification, examples of visualization with a multiphoton laser microscope of actin/myosin striations, nuclei and myofibers of skeletal muscle after vital staining by coating curcumin onto fascia of a mouse.
FIG. 29 is a diagram showing, at high magnification, examples of visualization with a multiphoton laser microscope of structure of bright center and dark shell of secondary nodule of lymph node after vital staining by coating curcumin onto lymph node of a mouse.
FIG. 30 shows visualization with a multiphoton laser microscope of cell bodies of hippocampal neurons after intraperitoneal administration of curcumin to a mouse.
FIG. 31 shows visualization with a multiphoton laser microscope of cerebral cortex after intraperitoneal administration of curcumin to a mouse.
FIG. 32 shows visualization with a multiphoton laser microscope of cell bodies of neuronal cells and blood vessels in cerebellum after intraperitoneal administration of curcumin to a mouse.
FIG. 33A is a diagram of visualization with a multiphoton laser microscope of capillaries of retina *in vivo* after intraperitoneal administration of sulforhodamine 101 to a mouse. (A) As indicated by the figure, thick blood vessels and capillaries in retina are visualized. Besides, it is shown that red blood cells inside the capillaries are visualized as black disk-shaped shadows.
FIG. 33B is a diagram of visualization with a laser microscope of capillaries of retina *in vivo* after intraperitoneal administration of sulforhodamine 101 to a mouse. (B) is a diagram showing capillaries at high magnification (the left figure). The capillaries of retina are shown to be anastomosed in a loop (indicated by lines in the right figure).

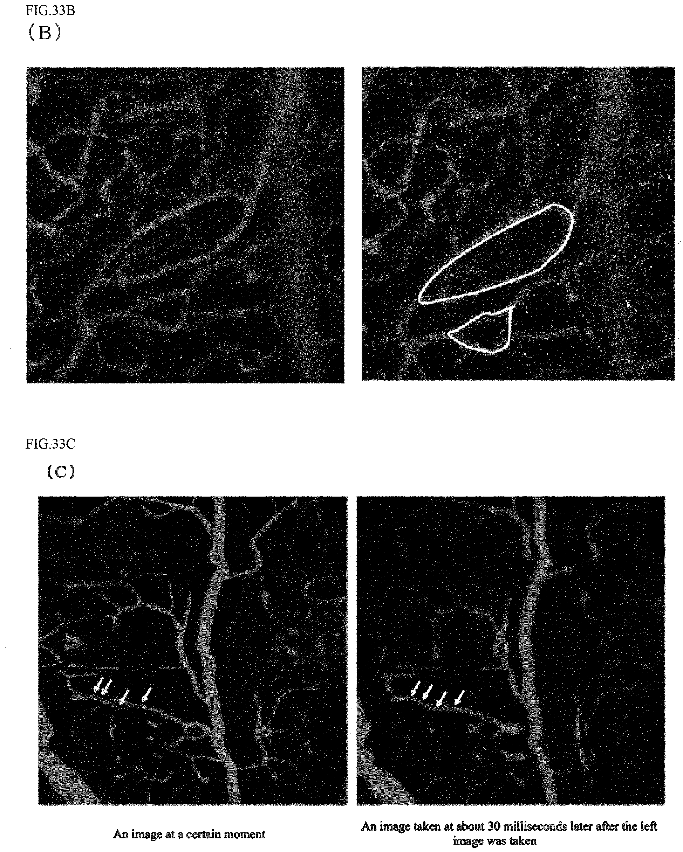

FIG. 33C is a diagram of visualization with a laser microscope of capillaries of retina *in vivo* after intraperitoneal administration of sulforhodamine 101 to a mouse. (C) is a diagram showing capillaries at high magnification. It is shown that red blood cells inside the capillaries are visualized as black disk-shaped shadows (indicated by arrows) (the left figure). The right figure shows a diagram obtained by photographing the same site again after about 30 milliseconds after photographing shown in the left figure. According to this figure, the number of red blood cells moving in the capillary and the moving speed of the red blood cells can be measured. Black disc-shaped shadows, which are red blood cells, are indicated by arrows.

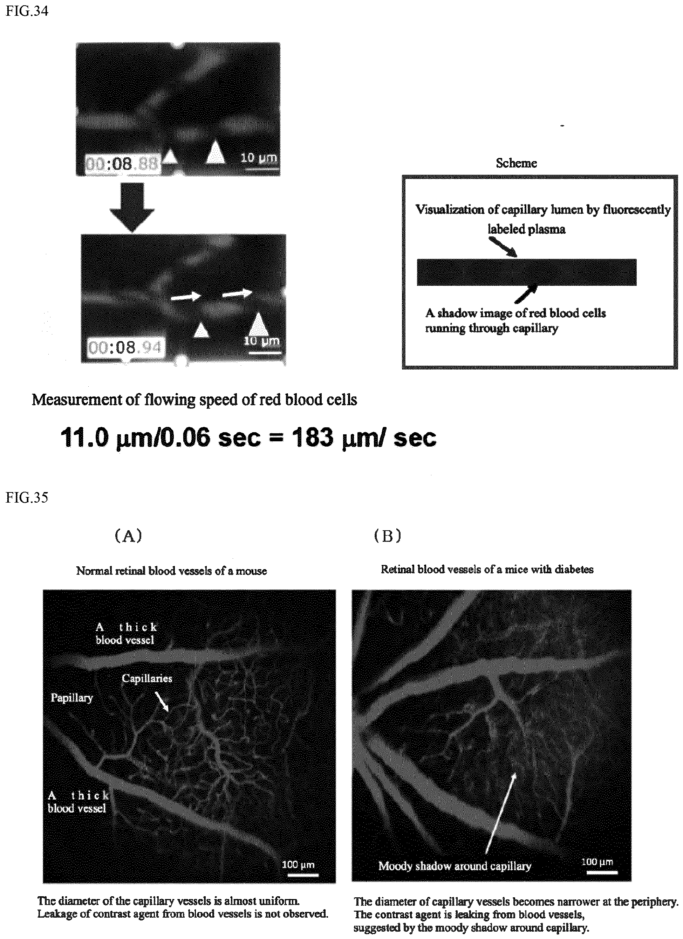

FIG. 34 shows the measurement of flowing speed of red blood cells by administering sulforhodamine 101 intraperitoneally into a mouse and visualizing the capillaries of retina *in vivo* by a laser microscope. The red blood cells moving in the capillary were photographed again 0.06 seconds later. The black disc-shaped shadows indicated by arrows are red blood cells. From the distance scale shown in the figure, the moving distance of the red blood cells during 0.06 seconds is 11.0 µm, and the moving speed is calculated to be183 µm/second. The schematic diagram shows the state in which red blood cells moving in the capillary are visualized.
FIG. 35 is a diagram of visualization with a multiphoton laser microscope of *in vivo* retinal blood vessels after intraperitoneal administration of sulforhodamine 101 to a normal mouse and a model mouse with diabetes. (A) shows retinal blood vessels of the normal mouse. The diameter of the capillary vessels is almost uniform, and the leakage from the blood vessels of sulforhodamine 101, a contrast agent, is not observed. (B) shows retinal blood vessels of the model mouse with diabetes. The diameter of the capillary vessels becomes narrower at the periphery. The leakage from the blood vessels of sulforhodamine 101 is observed due to the presence of mist-like signal around the blood vessels.

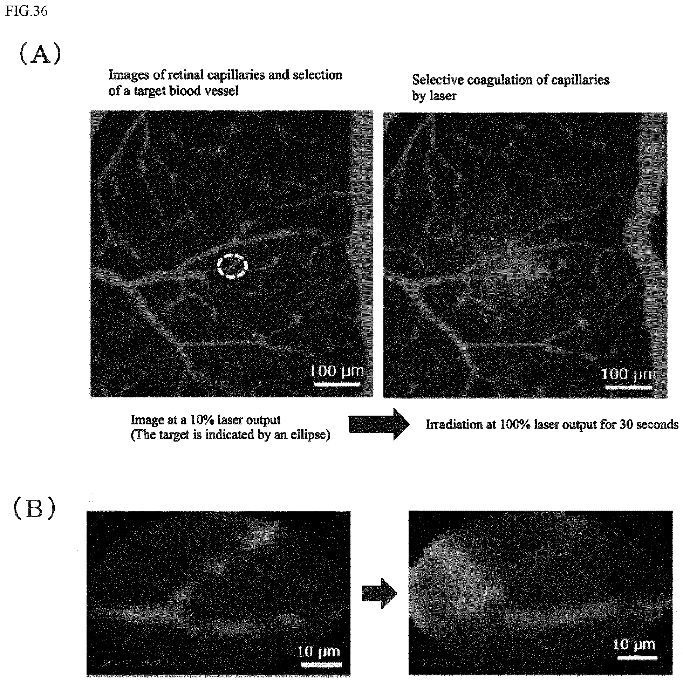

FIG. 36 shows coagulation of retinal capillaries in a mouse by multiphoton laser microscope. (A) Sulforhodamine 101 was intraperitoneally administered to a mouse. Retinal capillaries were visualized *in vivo* by a laser microscope at a 10% laser output. A target blood vessel to be irradiated with laser was selected (indicated by a dashed line ellipse in the left figure). Next, the laser output was set to 100% and laser irradiated for 30 seconds (the right figure). In the right figure, selective coagulation of the capillary subjected to the laser irradiation was observed. (B) shows a magnified image of the capillary subjected to the laser irradiation (the left figure: before the laser irradiation; the right figure: after the laser irradiation).
FIG. 37 is a photograph taken by a by multiphoton laser microscope showing diagram of neuronal layers of retina *in vivo* after vital staining by intraperitoneal administration of curcumin to a mouse.

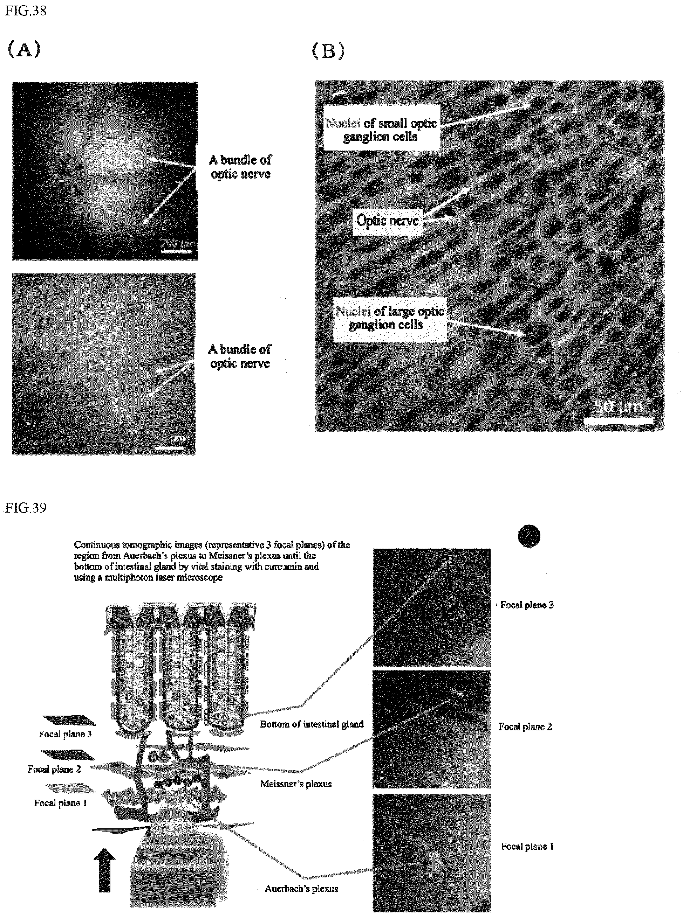

FIG. 38 is a diagram of visualization of optic nerves and the optic ganglion cells of retina *in vivo* after vital staining by intraperitoneal administration of sulforhodamine 101 and curcumin to a mouse. (A) is a diagram of visualization of blood vessels fluorescently labeled with sulforhodamine 101 and a bundle of optic nerve fluorescence-labeled with curcumin. (B) After vital staining by administering curcumin intraperitoneally into a mouse, the nuclei of the optic nerve, the small and large optic ganglion cells were visualized as negative signals (black round shadows), and their cytoplasm was visualized as positive signals.
FIG. 39 is a diagram showing continuous tomographic images (representative 3 focal planes) of the region from Auerbach's plexus to Meissner's plexus to the bottom of intestinal gland using a multiphoton laser microscope after vital staining with curcumin.
FIG. 40 is a diagram of visualization with a multiphoton laser microscope of site-dependent hypoplasia of Auerbach's plexus of large intestine in a patient with Hirschsprung-like disease after vital staining by administering curcumin to the 6-month old patient.
FIG. 41 is a diagram showing morphological characteristics of normal tissue and cancer in colonic epithelium and interface between cancer and normal tissue.
FIG. 42 is a diagram showing state of interface between cancer cells and normal tissue and cancer invasion.
FIG. 43 is a diagram showing state of interface between cancer cells and surrounding connective tissue and cancer invasion.

### EMBODIMENTS FOR CARRYING OUT THE INVENTION

Next, embodiments of the present invention will be described with reference to the figures. However, the technical scope of the present invention is not limited by these embodiments. The present invention can be implemented in various forms without changing its outline.

The "interface between a normal site and a lesion site", as used in the present invention means a boundary surface in which morphological abnormality of individual cells (cellular atypia) and abnormality in cell arrangement (structural atypia) in a lesion site are visible in relative to those of the cells in a normal site when a cell staining agent is used.

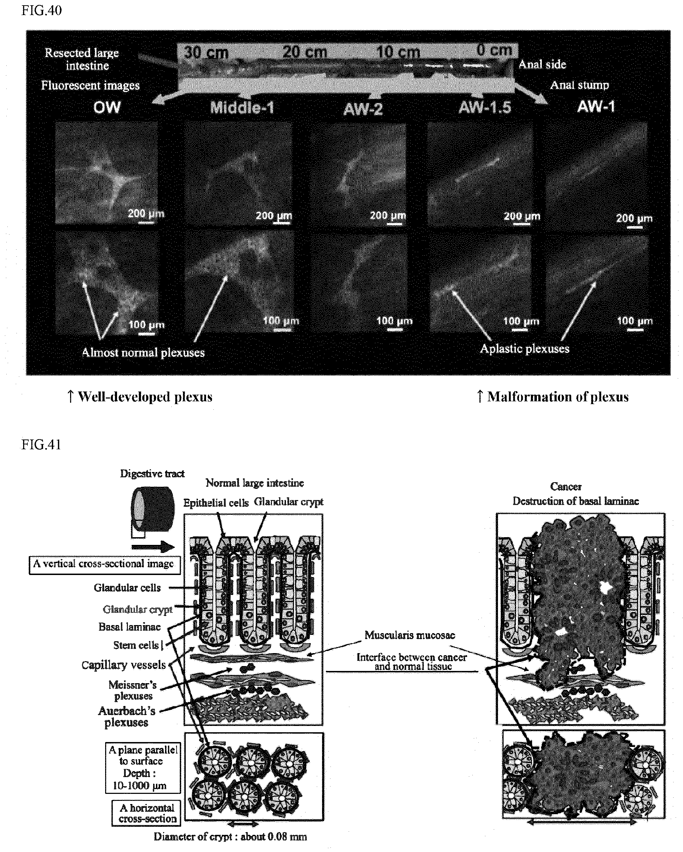

Cancer pathologic diagnosis is performed based on the morphological abnormality of individual cells (cellular atypia) and abnormality in cell arrangement (structural atypia). For example, in a normal case, the crypt of gland is uniformly distributed and regularly arranged as bottle-shaped structure in a vertical cross-sectional images and circumferential structure in a horizontal cross-sectional images. In a case of cancer, the crypt structure disappears. A surface where normal tissue having the uniformly distributed crypt structure is in contact with a group of cancer calls in different size can be defined as an interface (FIG. 41). In addition, cancer cells are generated near the stem cells of the crypt of gland and gradually proliferate. The case in which cancer cells have not yet exceeded mucosal muscle plates is defined as early-stage cancer, and the case in which cancer cells have already exceeded the mucosal muscle plates is defined as advanced cancer (FIG. 41).

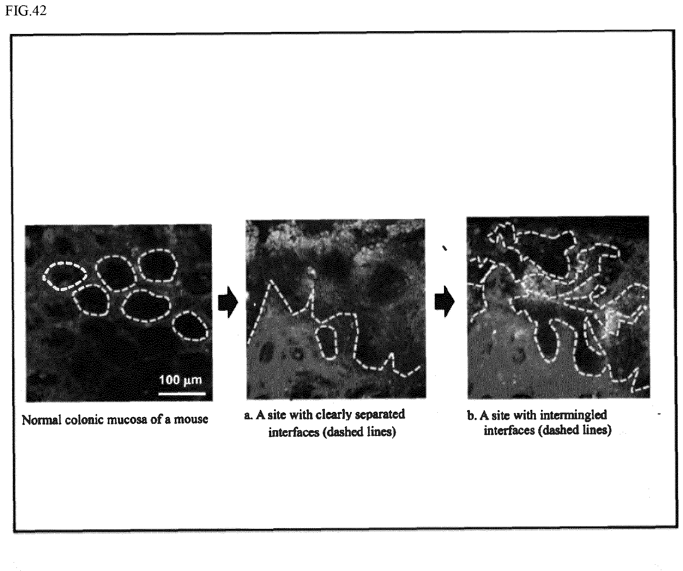

In addition, FIG. 42 shows an interface between cancer cells and normal tissue by single staining with curcumin. Comparing the cancer tissue existing in the visualized organ tissue with the crypt structure of the normal tissue, it can be confirmed in the cancer tissue that the regular crypt structure seen in normal tissue disappears, and a group of disorderly proliferating cancer cells without crypt structure is observed. Accordingly, the lesion site can be detected as cancer (FIG. 42).

It should be noted that cancer cells are generated near the stem cells of the crypt of gland and gradually proliferate. The case in which cancer cells have not yet exceeded mucosal muscle plates can be defined as early-stage cancer, and the case in which cancer cells have already exceeded the mucosal muscle plates can be defined as advanced cancer.

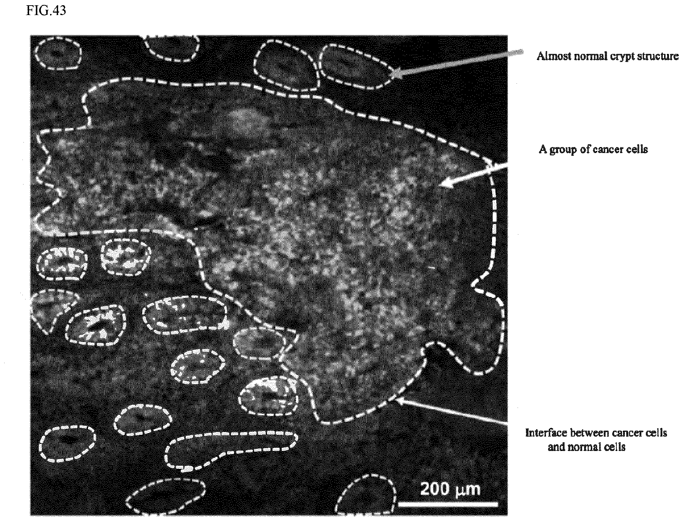

When vital staining a tissue with curcumin to stain epithelial cells/cancer cells and with Red #106 to stain connective tissue and capillaries, an interface can be defined as a boundary between epithelial cancer cells (in green) and connective tissue (in red). In this case, regarding the site where the interfaces (dashed lines) are clearly separated, cancer cells have not highly invaded their surroundings. The tumor with many such surrounding sites is determined to be less invasive. On the other hand, regarding the site where interfaces (dashed lines) are intermingled, cancer cells have highly invaded surrounding connective tissue. The tumor with many such surrounding sites is determined to be highly invasive (FIG. 43).

In normal colon mucosa of a mouse, since epithelial glands are in contact with surrounding connective tissue in crypt structure, the interface between them are in smooth circumferential shape. The crypt structure is uniformly distributed. Regarding the site where the interfaces (dashed lines) are clearly separated, cancer cells have not highly invaded their surroundings. The tumor with many such surrounding sites is determined to be less invasive. On the other hand, regarding the site where interfaces (dashed lines) are intermingled, cancer cells have highly invaded surrounding connective tissue. The tumor with many such surrounding sites is determined to be highly invasive (FIG. 43).

As described above, when using cell staining agents, by determining interfaces between normal sites and lesion sites, lesion sites can be determined, its progress can be monitored and appropriate methods for treatment and surgery can be provided according to early detection and progress of the disease.

Examples of the cell staining agents used in the present invention include vital staining agents consisting of one or more edible dye compounds. The dye compounds are selected from a group of fluorescent dyes including tar dyes (Red #3 (erythrosine), Red #104 (phloxine), Red #105, Red #106, Green #3 (Fast Green FCF), Red #2, Red #102, Blue #2 (indigo carmine), Yellow #4 (tartrazine), Yellow #5 (Sunset Yellow FCF) etc.), iridoid dyes (Haimeron P-2 (Gardenia Blue: geniposide), HI BLUE AT (Gardenia Blue dye: geniposide) etc.), carotenoid-based dyes (Haimeron P-2 (yellow dye: crocin), annatto (annatto N2R25, achiote fruit: bixin, norbixin), Haimeron P-2 (Gardenia Blue: geniposide), crocin G150 (Gardenia Yellow dye), crocin L (Gardenia Yellow dye), β-carotene, annatto WA-20 (annatto dyes, achiote seeds: norbixin) etc.), flavonoid-based dyes (HI RED G150 (grape peel dye, anthocyanin), HI RED RA200 (red radish dye: pelargonidin acyl glucoside), HI RED V80 (purple potato dye: cyanidin acyl glucoside and peonidin acyl glucoside), apigeninidine (kaoliang dye), cyanidin, delphinidin (eggplant dye), fisetinidine (*Acacia mearnsii* dye), malvidin (blue sweet pea dye), pelargonidin, robinetinidine (*Robinia pseudocacia* tree pigment), tricetinidine (black tea dye), petunidin (red berry dye), capsanthin (*capsicum* dye), epigallocatechin gallate, green tea, Safflower Y1500 (safflower dye, safflomin A+B), curcumin, sulfuretin, myricetin (grape, onion dye), or quercetin (onions, citrus dyes)), quinoid-based dyes (cochineal (Cochineal Red AL, carminic acid), HI RED S (lac dye/laccaic acid) etc.), betalain-based dyes (HI RED BL (red beet dye: betanin, isobetanin) etc.), indocyanine green and gingerol (ginger spicy ingredient), angiographic dye sulforhodamine 101 (Sigma, 5 mg/kg), angiographic dye fluoressein (Tokyo Kasei, 5 mg/kg), and Dye STK833131 (Vitas-M, 1∼25 mg/kg) that stains neurons.

Preferred examples of the cell staining agents used in the present invention include one or more staining agents selected from the group consisting of curcumin, sulforhodamine 101, angiographic dye fluolesein, STK833131, sulfretin, Red #3 (erythrosine), and Red #106.

Methods for administering cell staining agents are not particularly limited. For example, a cell staining agent of the present invention may be administered directly into the lumen of an organ or administered submucosally, or may be administered from serosal side of an organ. As methods for administering, coating, dropping or spraying can be adopted. Furthermore, as methods for administering cell staining agents, oral, intravenous, intraperitoneal, intrathoracic, or intrathecal administration can also be used. The administration method can be selected depending on the organ or site of the organ to be stained. When the stain has weak stainability, the mucosal surface can be treated with Pronase to remove mucus to improve the visibility of cell structure. When a staining agent is to be applied directly to the inner surface of a lumen (for example, by coating or spraying), the dosage form of the staining agent is preferably liquid, however, forms such as granules, tablets, or the like may also be used. Besides, appropriate additional components, for example, additives such as isotonizing agents, pH regulators, stabilizers, thickening agents, antiseptic agents, aromatics, or adhesives may be combined with a staining agent depending on the dosage form and other factors. For example, Pronase may be added previously to a staining agent of the present invention.

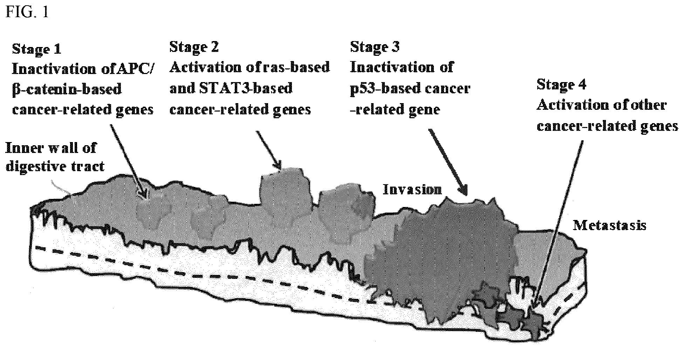

FIG. 1 is a schematic diagram showing a stepwise process of malignant transformation of a living cell group on the surface of inner wall of digestive tract. In FIG. 1, the process of malignant transformation of a living cell group is shown in the order from stage 1, stage 2, stage 3, to stage 4.

At stage 1, malignant transformation starts in part of a living cell group. It is considered that stage 1 occurs when activity of APC/β-catenin-based cancer-related gene is weakened and the function of suppressing cell proliferation is reduced. At this stage, the proliferation of cells is slightly enhanced, indicating that at least a precancerous state that the cells can become cancer cells in the future is expressed.

The stage 2 is a precancerous state in which cancer has progressed more than stage 1. It is considered that the activity of ras-based cancer-related gene is enhanced and cell proliferation is enhanced in stage 2. It is also considered that STAT3-based cancer-related genes may be activated at this stage. The size of a cancer cell group is small, and its diameter is, for example, 0.1∼0.4 mm. The diameter of a cancer cell group is that of a circle having the same area of the cancer cell group when it is regarded as a circle. This stage is not immediately life threatening for a patient. However, it is desirable to make a plan for treatment etc. for the future.

At stage 3, part of a living cell group is invading, and cancer cells are revealed. It is considered that stage 3 occurs when the activity of p53-based cancer-related genes is weakened and the function of suppressing cell proliferation is reduced. At this stage, the activities of both p53-based and APC/β-catenin-based tumor suppressor gene products are weakened, and the function of suppressing cell proliferation is greatly reduced. Therefore, the proliferation of cancer cells accelerates, and the cancer cells invade surrounding tissue. When arriving at stage 3, the diameter of a cancer cell group reaches 0.5 mm or more, and if left as it is, the cancer that induces death of an individual is completed.

At stage 4, cancer cells completed in stage 3 have become cancerous, further cause genetic mutations, and have progressed to malignant cancer susceptible to cell proliferation, invasion and metastasis. At this stage, cancer metastasis to other distant organs other than digestive tract begins, which is life-threatening and dangerous. It is considered that the speed of progress from stage 1 to stage 4 depends on the activation state of cancer-related genes.

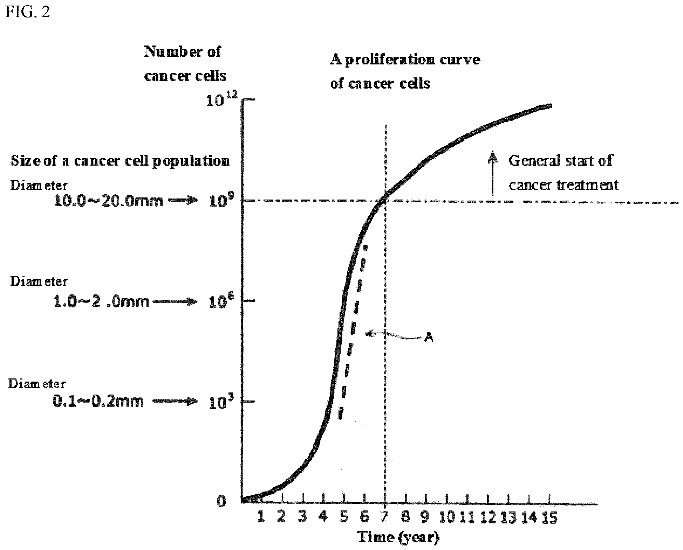

FIG. 2 is a diagram showing an example of a proliferation curve of human cancer cells. As shown in FIG. 2, generally, the number of cancer cells increases according to a predetermined proliferation curve. For example, the slope of the proliferation curve is small during the first 3 years when malignant transformation is about to begin (a period when the diameter of a cancer cell group is less than 0.2 mm), however, it turns large after 4 years (a period when the diameter of a cancer cell group is 0.5 mm or more), and decreases slightly after 7.5 years. Generally, it is after 7 years when cancer is clinically detected and treated. This is because a cancer cell group cannot be detected unless its diameter reaches 10 mm or more. Currently, cancers are generally detected after their diameter exceeds 20 mm. Accordingly, it is commonly treated by removing cancer cells through surgical resection.

Regarding FIG. 2, it should be noted that the size of a cancer cell group increases exponentially in the extent indicated by the dashed line A in the proliferation curve of FIG. 2. This exponential increase indicates that cancer cells in a cancer cell group have completed gene mutation in stages 1∼3 of cancer which should occur, and cancer cells are dividing repeatedly at a certain constant speed. In the early stages of this exponential increase, that is, when the cancer-related gene expression pattern is abnormal, but the cancer cell group itself is as small as 1 mm or less in diameter. If these cancer cell groups (ultra-early cancer) can be detected, the cancer can be cured radically since these ultra-early cancers are small enough to be completely and easily removed. In this way, at the ultra-early stage, if the malignancy of malignant transformation can be grasped as abnormality in the expression pattern of cancer-related genes, cancer can be treated radically before reaching dangerous stages.

In order to detect ultra-early cancers, the inventors have tried to determine malignancy of malignant transformation by imaging cancer-related gene expression pattern of a living cell group with a multiphoton laser microscope or a confocal laser microscope, and visualizing the activation state of cancer-related genes.

For the visualization of expression patterns of cancer-related genes in living cells, the inventors stained a cancer-related gene product to chromatic color using staining agents containing edible dyes and photographed. An edible dye is a natural or artificial dye which has been permitted to be administered to human (For example, a dye for food coloring, or a dye that can be taken as supplements).

Specifically, a staining agent containing curcumins (C₂₁H₂₀O₆) can be used to selectively stain a STAT3-based cancer-related gene product. In addition, a staining agent containing Red #3 (erythrosine) can be used to selectively stain the expression pattern of ras-based cancer-related gene.

More specifically, as a staining agent containing curcumins, a solution containing 1% by weight of curcumin was prepared. As a staining agent containing Red #3, a solution containing 1% by weight of phloxine was prepared. As a staining agent containing curcumins, a curcumin solution 5∼100-fold diluted from its stock solution (for example, a liquid containing 5% curcumin solution, 45% glycerol, and 50% ethanol) with physiological saline can be used. As a staining agent containing 1% Red #3, a phloxine solution at the concentration of 10 mg/mL (stock solution) or its diluted solution (up to 10-fold) can be used.
(i) A solution prepared by diluting chemically synthesized curcumin to about 1 mg/ml with a solution containing 0.45% glycerin and 0.5% ethanol is used.
(ii) A solution prepared by dissolving 1 g of Okinawa curcumin powder in 10 ml of PBS and diluting to about 1 mg/ml is used.

Both (i) and (ii) are sterilized with sterilizing filters immediately before biogenic administration.

When a staining agent containing curcumins is used, the expression of a STAT3-based cancer-related gene product in living cells can be visualized by staining. In addition, when a staining agent containing Red #3 is used, the expression of ras-based cancer-related genes in living cells can be visualized by staining. After the staining, excess staining agent can be removed by rising. Excess staining agent can be removed by performing rising for about 10 seconds for 3 times with a physiological solution such as physiological saline or phosphate buffered saline that does not damage cells or biological tissue. When performing double staining using different staining agents, it becomes possible to simultaneously analyze the expression levels of STAT3-based and ras-based cancer-related gene products. The staining time of each staining agent can be 1∼5 minutes. At the above-mentioned concentration, it does not penetrate into the nucleus in a cell within 10 minutes from the start of staining, even though it penetrates into cytoplasm. Accordingly, the nucleus surrounded by the cytoplasm is clearly visualized, making it clearer to be analyzed. The staining times (duration from administration to observation) are, for example, 1∼5 hours in case of coating directly to mucosal or organ surface, 1∼5 hours in case of oral administration, 3 minutes - 1 hour in case of intravenous administration, 3 minutes - 5 hours in case of intraperitoneal administration, 3 minutes - 1 hour in case of subcutaneous injection, 3 minutes - 2 hours in case of intramuscular injection, 5∼30 minutes in case of intraorgan injection, and 3 minutes - 5 hours in case of intrathoracic or subarachnoid administration. However, they are not limited by these examples. In order to enable visualization of a lesion in an organ, a staining agent may be administered to the organ in a sufficient period and amount according to the purpose. For example, in the case where the large intestine mucosa is stained with curcumin or Red #106 as cell staining agents, the staining agent penetrates from the mucosal surface to the inside of about 50 µm within 3 minutes of staining time, however, when the staining time is extended to about 60 minutes, the cell staining agent penetrates into the tissue with a diameter of about 1 mm, and the tissue can be dyed. In order to dye the tissue in deeper part, the staining time may be extended up to 5 hours.

The staining of cancer cells with the above-mentioned cell staining agents can be performed directly on organs. Organs derived from human or animals can be used. The organ to be stained may be an extirpated organ or an *in-vivo* organ. Examples of organs include, but are not limited to, large intestine, lung, prostate, stomach, esophagus, bladder, lymph nodes, and the like. In the case of staining a lymph node, it is preferable to apply a staining solution after exfoliating the surface tissue covering a lymph node tissue in order to increase the permeability of a staining solution. Cells stained with a cell staining agent can be imaged using a multiphoton laser microscope or a confocal laser microscope. When multiphoton laser is used, the wavelength of laser is preferably 600∼1600 nm in order to achieve a sufficient imaging depth and resolution from an organ surface. When confocal laser is used, the wavelength of laser is preferably 400∼700 nm.

The application of a staining solution to an organ can be performed from serosal side covering the organ surface. For tubular organs such as large intestine, stomach, and esophagus, it can also be performed from lumen side. Applying means coating, dropping or spraying a cell staining solution onto an organ. For the purpose of pathological diagnosis of an organ resected after a surgery, if a removed organ is tubular, tissue staining can be performed from luminal side. However, in order to identify a resection site or a cancer invasion site during surgery, it is desirable to perform tissue staining from serosal side of an organ. This is because, for example, robot technology for endoscopic surgery on the abdominal cavity or the like basically performs a surgery from serosal side of an organ, and thus it requires tissue staining on the serosal side. When a staining solution is applied from serosal side, a sterilized staining solution is coated, dropped or sprayed to an organ serosa in the surgical field, and within 10 minutes after the application of the staining solution, preferably 1∼5 minutes, and more preferably 1~3 minutes, the organ is rinsed with physiological saline or the like, and the staining solution is removed. Immediately thereafter, stained images can be observed with a multiphoton laser microscope or a confocal laser microscope. In addition, a staining agent can be administered systemically before a surgery, and a tissue can be observed with a laser microscope during the surgery. Oral or intravenous administration can be used as an administration method.

Regarding staining an organ tissue, the object in the organ to be visualized differs depending on the staining agent used. For example, curcumin and Red #3 are suitable for staining epithelial and glandular cells, as well as cancerous cells derived from them. On the other hand, Red #106 is suitable for staining connective tissue and capillaries. By laser irradiation, curcumin gives a green fluorescent color, while Red #3 and Red #106 give a red fluorescent color. Therefore, double staining with curcumin and Red #106 makes it easier to identify structure of cells in tissue by superimposing stained images. Accordingly, micro cancer tissue with diameter of about 1 mm and invasive cancer cell groups can be detected.

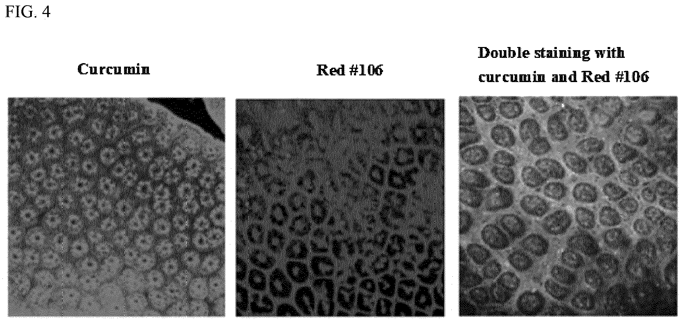

FIG. 4 shows isolated large intestine of a mouse (normal mucosal tissue) vital-stained with curcumin and Red #106 from lumen side and photographed with a multiphoton laser microscope from the lumen side. As a result, curcumin stains cytoplasm of epithelial and glandular cells, and Red #106 stains connective tissue, capillaries and cell membranes of epithelial and glandular cells. Accordingly, it is indicated that structures of cells in tissue can be clearly identified by double staining.

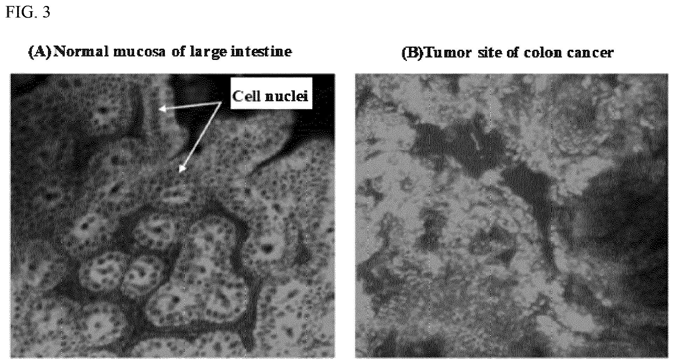

It was found out by tissue staining that observation images with laser microscope of normal tissue without cancer cells and those of tumor tissue with cancer cells are different. For example, when mucosa of large intestine is stained with curcumin (see FIG. 3), in normal mucosal tissue of large intestine, the cytoplasm of epithelial cells and gland cells is stained, but the nucleus is not stained. Thus, morphology of individual cells and nuclei is clearly visible. On the other hand, in the tissue of a tumor site of colon cancer, the size of each cell is uneven, the nucleus is large, and the arrangement or sequence of the cells is uneven. Further, dissociation of cell adhesion is observed, which is detected as cellular atypia. Moreover, in cancer tissue, cell groups are not aligned and arranged on basement membrane and do not form glandular structures, which is detected as structural atypia. According to the method of the present invention, cancer cells can be detected, that is, pathological diagnosis of cancer can be performed based on images which indicate the differences between normal tissue and cancer tissue as described above.

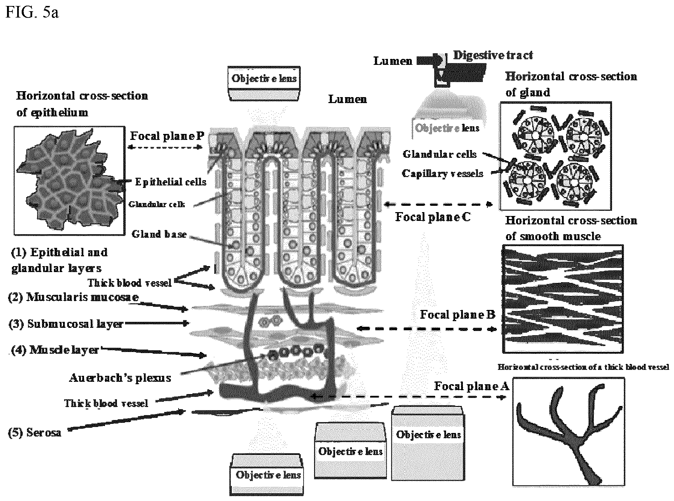

Further, invasion of cancer cells can be detected in relation to five-layer structure of normal large intestine. The tissue structure of normal large intestine is shown in FIG. 5a, and the five-layer structure is shown as (1) to (5). When imaging with a confocal laser or a multiphoton laser microscopic endoscope, normal large intestine consists of five layers, which are epithelial and glandular layers (1), muscularis mucosae (2), submucosal layer (3), muscle layer (4), and serosa (5) in the order from the surface facing lumen through which food passes towards deep parts.

The surface facing the lumen is covered all over by the epithelial and glandular layers (1). The epithelial cells form glandular structures that are vertically invaginated from the surface in an bottle shape (The vertically invaginated structures are also called crypt structures.) at certain intervals. The epithelium looks like a sheet of cells in which epithelial cells are tightly aggregated, as shown by focal plane P in FIG. 5a. On the other hand, as shown by focal plane C in FIG. 5a, the glandular structure is in a shape in which about 10 gland cells are arranged concentrically toward the central aperture, and capillaries having a diameter of about 10 µm are surrounded around the outside thereof. The height of gland is about 0.5∼1.0 mm, and its part at one third of the depth is called gland base. The cells in this part divide and renew gland cells and epithelial cells. It is considered that cancer is caused by abnormally enhanced division of cells at the gland base. The muscularis mucosae (2) is a layer of thin smooth muscle present deep in the glandular structure. When cancer has not developed beyond the muscularis mucosae, it is called an early-stage cancer. The submucosal layer (3) is a layer of loose connective tissue. The muscle layer (4) is a layer of thick smooth muscle that governs intestinal peristalsis. (As shown by focal plane B, it has been found out that this layer contains a group of elongated smooth muscle cells.) Inside this smooth muscle layer, a network of autonomic nerves controlling the movement of this smooth muscle is distributed, which is called Auerbach's plexus. Furthermore, inside the muscle layer, there are also thick blood vessels that supply blood to capillaries around epithelia and glands. (As shown by focal plane A, thick blood vessels with a diameter of 20 µm or more are observed in this layer.) The serosa (5) is a layer consisting of flat cells.

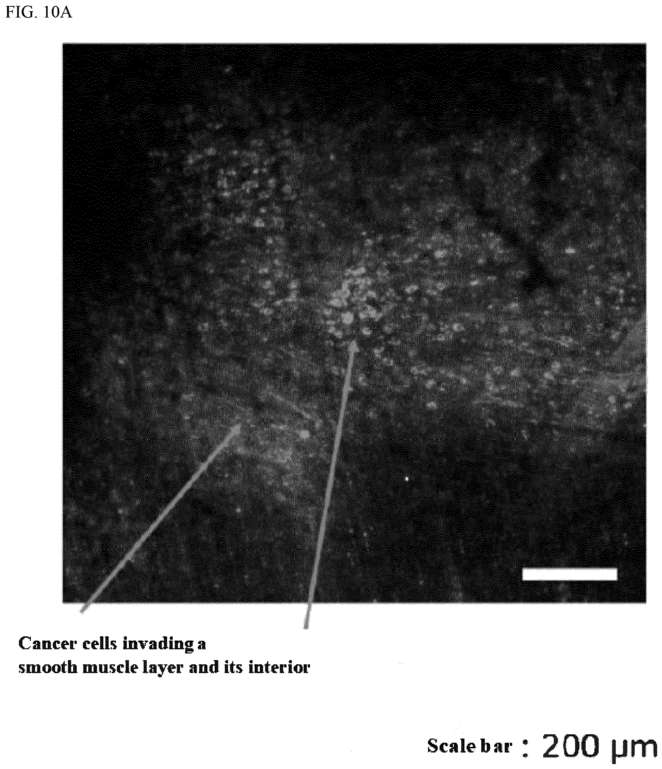

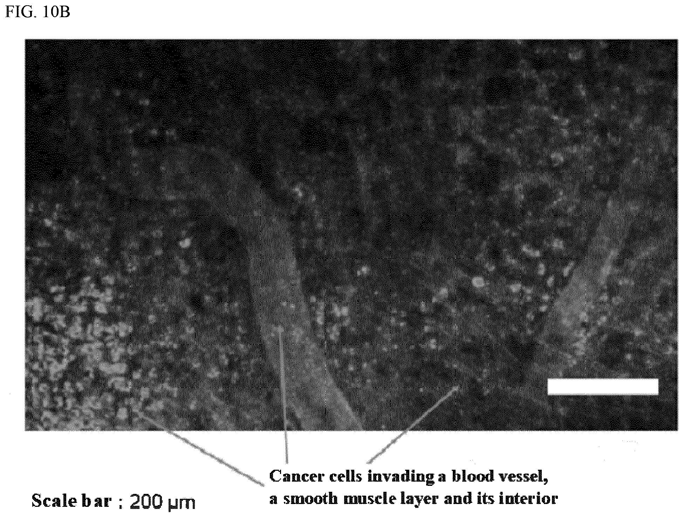

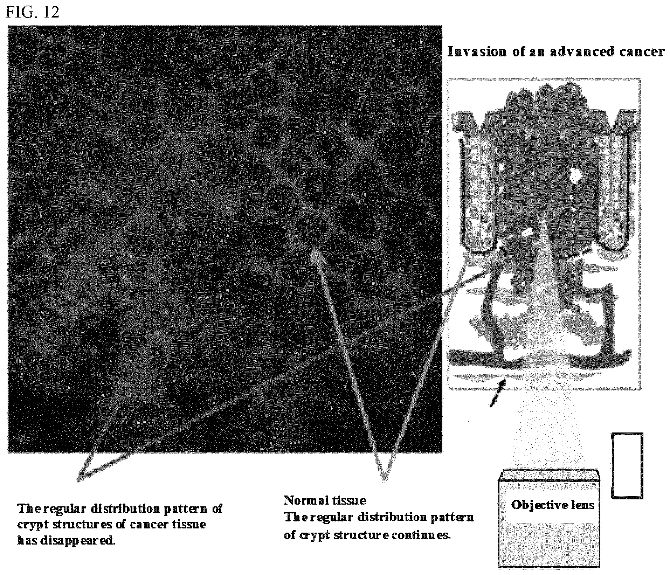

It was found out that among the above five-layer structure, curcumin stains epithelial and glandular cells in epithelial and glandular layers (1) strongly positively, the smooth muscle in muscularis mucosae (2) moderately positively, the smooth muscle in muscle layer (4) slightly positively, and Auerbach's plexus inside muscle layer strongly positively, while Red #106 stains a network of capillaries surrounding the glandular structures of epithelial and glandular layers (1) strongly positively, the smooth muscle in muscularis mucosae (2) slightly positively, the smooth muscle in muscle layer (4) slightly positively, and the wall of a thick blood vessel inside muscle layer strongly positively. The visualization of the five-layer structure of normal large intestine and major cell structures by these vital staining agents is a very useful clue in determining the extent of cancer invasion. That is, whether or not there is cancer invasion can be determined accurately by combinations of the finding that the above normal structure does not exist at a normal position with a normal distribution pattern (disappearance of the regular distribution of crypt structures at the gland base by Red #106 at the cancer site, FIG. 12) and the finding that there are cells that should not be present in places where they should not be present in normal cases (many large cells positive for curcumin staining are chronically scattered inside smooth muscle layer. See FIG. 10A and FIG. 10B).

Since it only takes a short time to perform the procedures from staining of organ tissue to observing with a laser microscope, pathological diagnosis of cancer can be applied to pathological diagnosis *in-vivo* during surgical operations. In general, pathological diagnosis of cancer is performed based on the difference in size and shape of cells (cellular atypia) and the disorder in structure of tissue (structural atypia). Those with severe atypia are detected to be cancer (malignant), and those with mild atypia are detected to be benign.

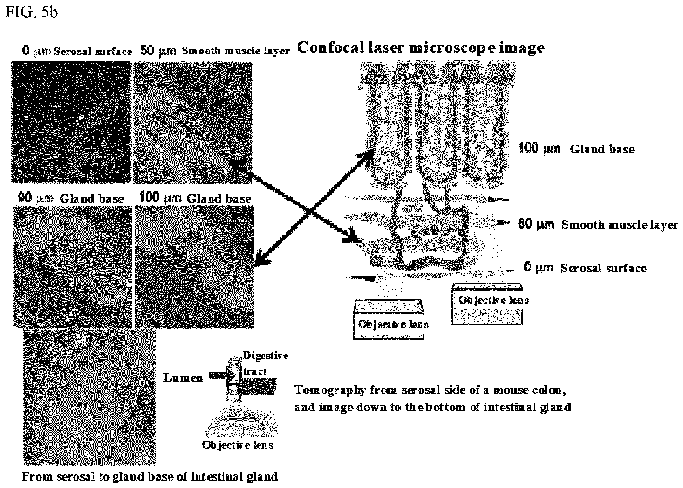

In tissue observation with a laser microscope, the focal plane with respect to an organ can be changed by manipulating the position of objective lens of the laser microscope. By this procedure, cell morphology from an organ surface to a depth of 0.05∼1.0 mm can be clearly observed as tomographic images. For example, in case of observing the tissue of large intestine from serosa side, when the depth of focus is changed sequentially from serosa toward lumen, relatively thick blood vessels which are close to the serosa, the smooth muscle layer, the Auerbach's plexus which is located inside and controls the movement of smooth muscle, and then the glandular structure including capillaries can be observed. By observing a smooth muscle layer, even cancer cells that have invaded into smooth muscle layer can be detected. FIG. 5b shows the results of staining an isolated large intestine (normal mucosal tissue) of a mouse with curcumin from luminal side and photographing with a multiphoton laser microscope while changing the focal length from the serosal side. In the tissue structure of large intestine described above, the surface of serosa (5) can be observed clearly at the focal length of 0 µm; the smooth muscle of muscle layer (4) can be observed clearly at the focal length of about 50 µm; and the glandular structure (crypt structure) of epithelial and glandular layers (1) can be observed clearly at a focal length of 80∼160 µm in mice.

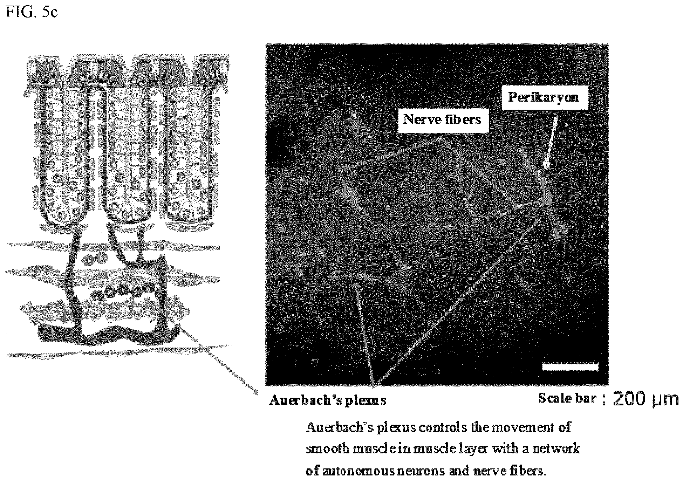

In an embodiment, plexus can be visualized by using the method of the present invention. FIG. 5c is an image obtained by vital staining an isolated large intestine (normal mucosal tissue) of a mouse to from serosa side with curcumin and observing it with a multiphoton laser microscope. As indicated by the figure, Auerbach's plexus in muscle layer is stained positively. That is, it is understood that curcumin visualizes Auerbach's plexus as a network-like structure because it stains perikaryon strongly, while stains nerve fibers moderately. The Auerbach's plexus belongs to autonomic nervous system and consists of perikaryon of neurons and nerve fibers that connect them.

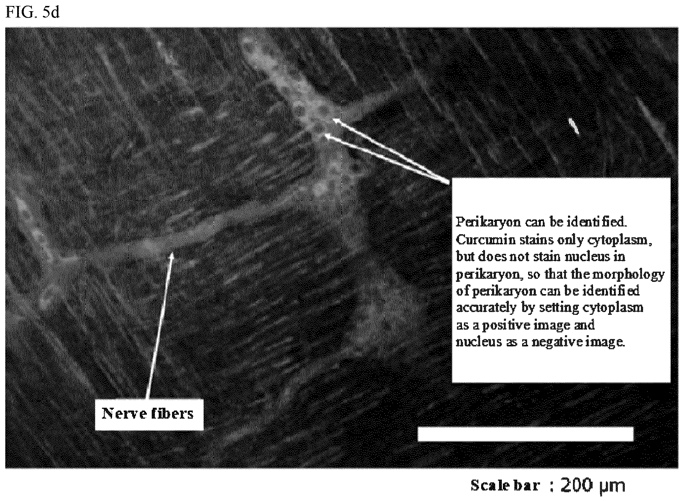

FIG. 5d shows an image, at high magnification, of the above-mentioned Auerbach's plexus in muscle layer observed with a multiphoton laser microscope. As indicated by the figure, perikaryon can also be identified. Regarding the perikaryon, curcumin stains only the cytoplasm and does not stain the nucleus, so that cytoplasm is recognized as a positive image and nucleus is recognized as a negative image. Thereby, the morphology of perikaryon can be accurately determined. Cancer cells develop in epithelial and glandular layers (1) (see FIG. 5a), then spread to other layers, and migrate (a phenomenon called cancer cell invasion). It is known that invading cancer cells tend to move along blood vessels and peripheral nerves. However, the ability to visualize Auerbach's plexus in muscle layer by vital staining with curcumin results in visualization of cancer invasion pathways, which is useful in determining the extent of cancer invasion.

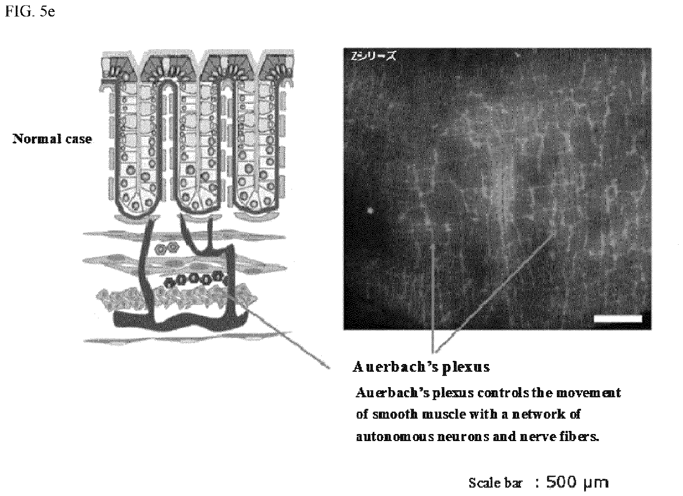

In an embodiment of the present invention, Auerbach's plexus in muscle layer can be observed with a multiphoton laser microscope. FIG. 5e is a diagram in which Auerbach's plexus in a muscle layer was visualized by staining with curcumin. In the multiphoton laser microscope image, many tomographic images can be reconstructed by z-stacks of serial optical sections and superimposed, so that the network structure of Auerbach's plexus can be visualized in a wider extent.

In an embodiment of the present invention, curcumin was administered to a freshly resected colon from a patient with Hirschsprung-like disease, and then a multiphoton laser microscope was used to visualize Auerbach's plexus. The normal part and malformation part of the Auerbach's plexus in large intestine of the patient were clearly identified and the first in human case was accomplished. (FIG. 39). Therefore, intraoperative rapid diagnosis by a multiphoton laser microscope of freshly resected large intestine of a patient with Hirschsprung-like disease can be performed and appropriate surgical resection sites can be confirmed.

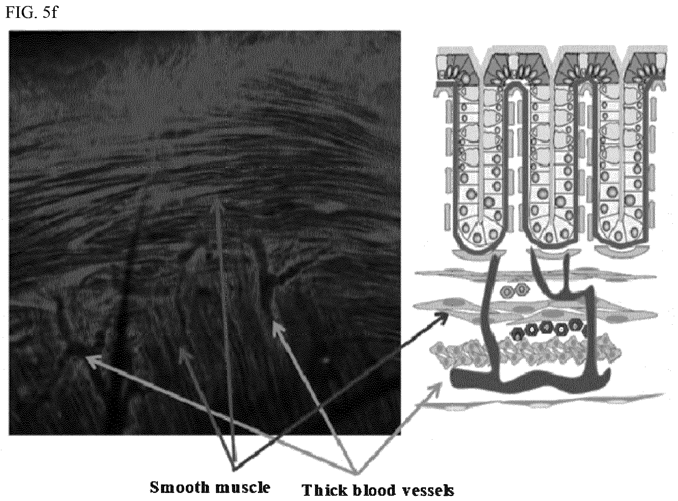

In an embodiment of the present invention, thick blood vessels and smooth muscle can be visualized by vital staining with Red #106 in FIG. 5f. A stained isolated large intestine (normal mucosal tissue) of a mouse was photographed from serosal side with a multiphoton laser microscopy by focusing on the muscle layer (4) (see FIG. 5a). It is shown that the smooth muscle and vessel walls are strongly stained.

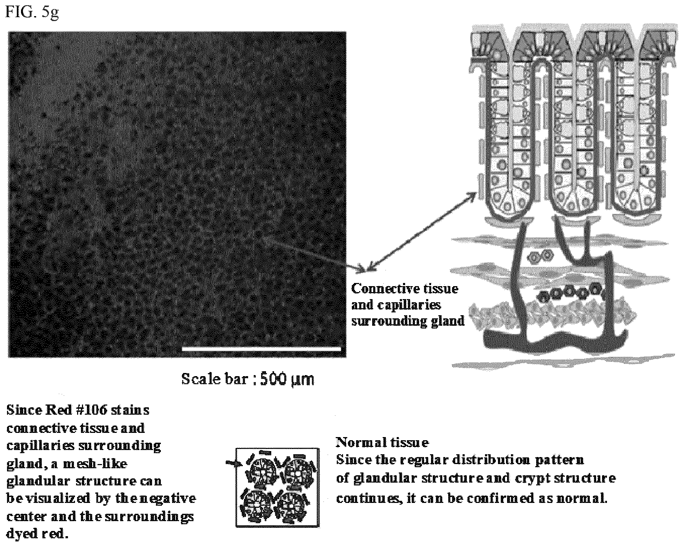

In an embodiment of the present invention, a tissue stained with Red #106 was imaged from serosal side with a multiphoton laser microscope. Structures of gland and crypt can be visualized. FIG. 5g was taken by focusing on epithelial and glandular layers (1) (see FIG. 5a). From the image obtained, because the connective tissue and capillaries are stretching around the gland cells in a circumferential pattern, the distribution pattern of structures of gland and crypt is visualized. This regular distribution pattern of structures of gland and crypt is a major feature of tissue structure of normal large intestine. When cancer occurs, this regularity of the structures of gland and crypt at a cancer site is lost.

As described above, when a normal mucosal tissue of large intestine of a mouse is imaged with a confocal laser and a multiphoton laser microscopic endoscope by the method of the present invention, it was found out that among the five-layer structure of normal large intestine, that is epithelial and glandular layers (1), muscularis mucosae (2), submucosal layer (3), muscle layer(4), and serosa (5), curcumin stains glandular cells in epithelial and glandular layers (1) strongly positively, the smooth muscle in muscularis mucosae (2) severely positively, the smooth muscle in muscle layer (4) slightly positively, Auerbach's plexus inside muscle layer strongly positively. While Red #106 stains a network structure of capillaries surrounding the glandular structures of epithelial and glandular layers (1) strongly positively, the smooth muscle in muscularis mucosae (2) moderately positively, the smooth muscle in muscle layer (4) strongly positively, and the wall of a thick blood vessel inside muscle layer strongly positively. The visualization of the five-layer structure and major cell structures of normal large intestine by these vital staining agents is a very useful clue for determining the extent of cancer invasion as described below. That is, presence of cancer can be determined by combinations of the finding that the above normal structure does not exist at normal sites and the finding that cells which do not exist in the case of normal structure exist.

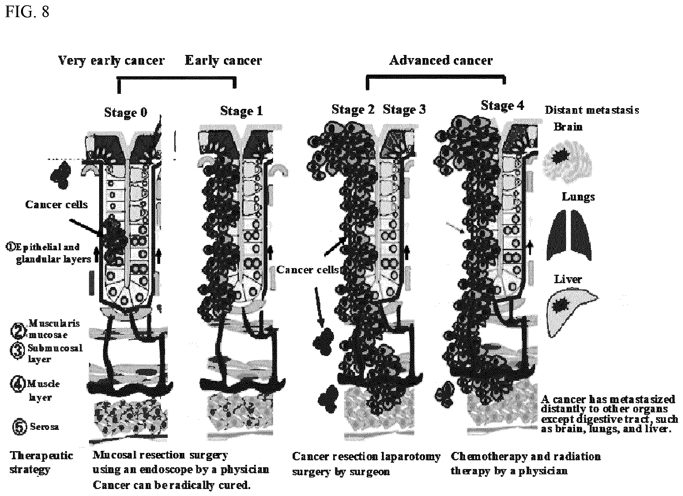

The determination of degrees of cancer progression due to local invasion and metastasis and the treatment strategy will be described with reference to FIG 8. Cancer is generally considered to be originated from the cells located at gland base, and caused by stepwise gene mutation shown in FIG. 1 of undifferentiated cells that undergo cell division and proliferation even in a normal state, and abnormal enhancement of cell division and proliferation. The stage wherein cancer cells do not exit epithelial cells is defined as intraepithelial stage 0 or ultra-early stage of cancer. The stage wherein cancer cells proliferate beyond the area where epithelial cells originally exist in the area where they occurred, but do not cross the muscularis mucosae is defined as stage 1 or early stage of cancer. The stage wherein cancer cells invade the submucosal and muscle layers beyond the muscularis mucosae is defined as stages 2-3. The stage wherein cancer cells have spread to other organs beyond local tissue or organ where they occurred is defined as stage 4. In general, treatment methods mainly include removal of cancer tissue with endoscopy in a case of stages 0 to 1, removal of cancer tissue by surgery in a case of stages 2 to 3, and chemotherapy, radiation therapy and immunotherapy in a case of stage 4. When removing or resecting cancer tissue by surgery for a case of stage 2 or 3 cancer, the invasion area of cancer cells and the cancer tissue can be examined from the serosal side by using vital staining and laser endoscopy. The present invention is useful for presenting supportive image data to determine the appropriate cutting line to be removed or resected.

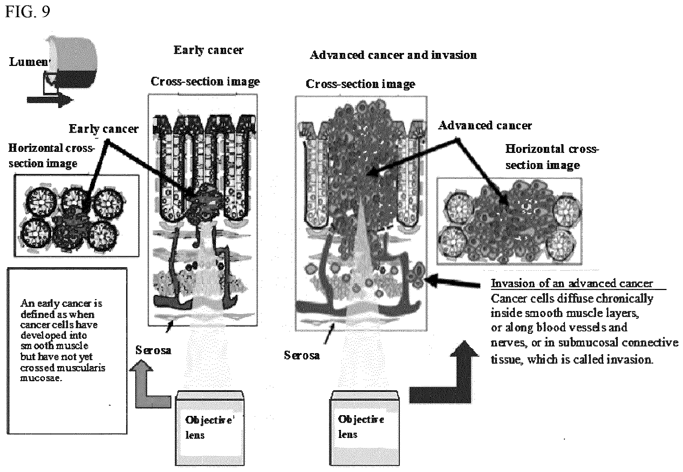

In a case of local invasion of advanced cancer, cancer cells diffuse chronically along blood vessels and nerves inside smooth muscle layers or in submucosal connective tissue (see FIG. 9). This phenomenon is called local invasion of cancer cells. It is extremely difficult to accurately determine the extent of local invasion by current methods such as close detection by naked eyes and change in the hardness of a tissue by palpation.

However, the presence of cancer cells can be clearly recognized by staining a cancer tumor site in a colon cancer mouse with curcumin from serosal side and observing from the serosal side with a multiphoton laser microscope. Referring to FIG. 10A, a large number of large cells positive for curcumin staining are scattered chronically inside the smooth muscle layer. These cells are determined to be invaded cancer cells. In addition, since the cytoplasm of these cells is darker than surrounding tissue by curcumin staining and recognized as a positive image, these cells are determined to be cancer cells. That is, these cells are determined to be cancer cells by the finding that cells that should not exist in a normal case exist in places where they should not exist. Referring to FIG. 10B, cancer cells invading blood vessels and a smooth muscle layer are observed. The fact that some cancer cells aggregate around blood vessels suggests that cancer cells have the character of moving along blood vessels and infiltrating.

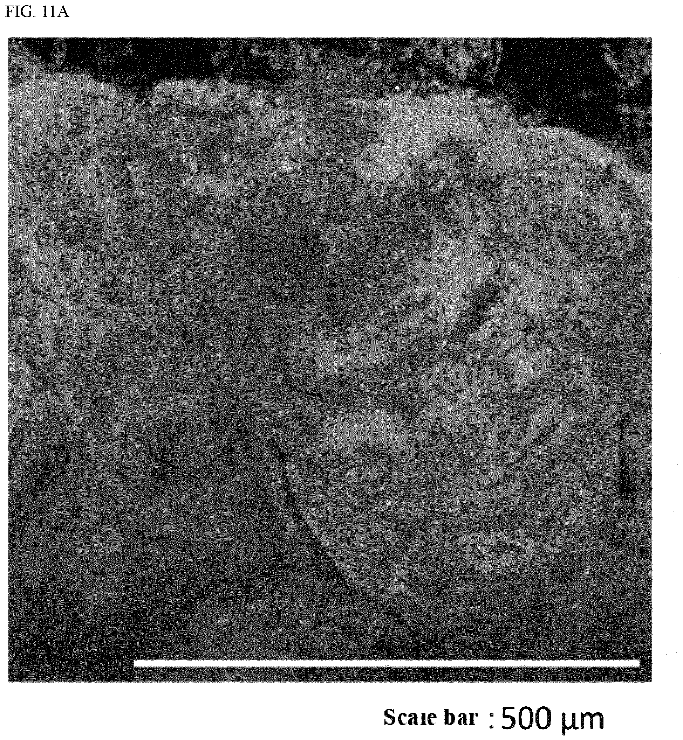

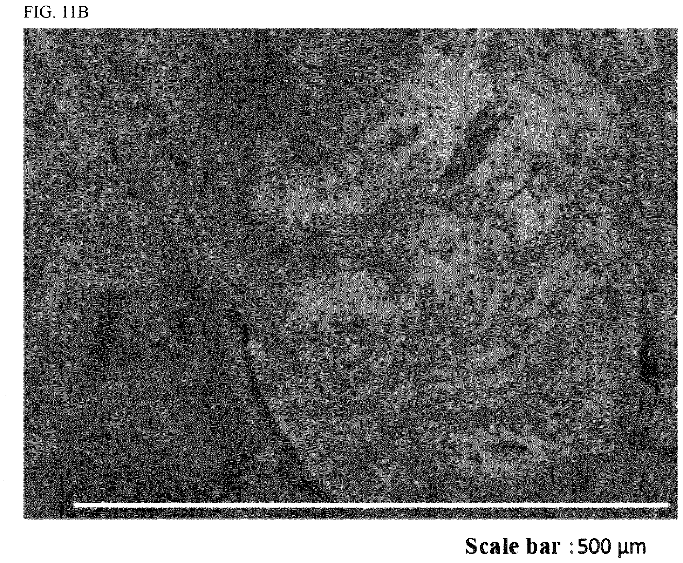

FIG. 11A and FIG. 11B are photographs taken from luminal side of a tumor site of a colon cancer mouse, after staining with curcumin from the luminal side. As indicated by these photographs, in cancer cells, cytoplasm is stained severely positively, while nuclei are stained negatively. As a result, cellular atypia and structural atypia of cancer can be distinguished.

FIG. 12 shows a photograph of a cancer tumor site of a colon cancer mouse taken from serosal side with a multiphoton laser microscope, after staining with Red #106 from the serosal side, and a schematic view of advanced cancer invasion. In normal tissue, it can be confirmed that the crypt structure is regularly distributed. While in cancer tissue, the regular distribution of crypt structure has disappeared, and the case can be determined as cancer. That is, it is determined that cancer cells exist at this site because a normal structure is not at its normal site.

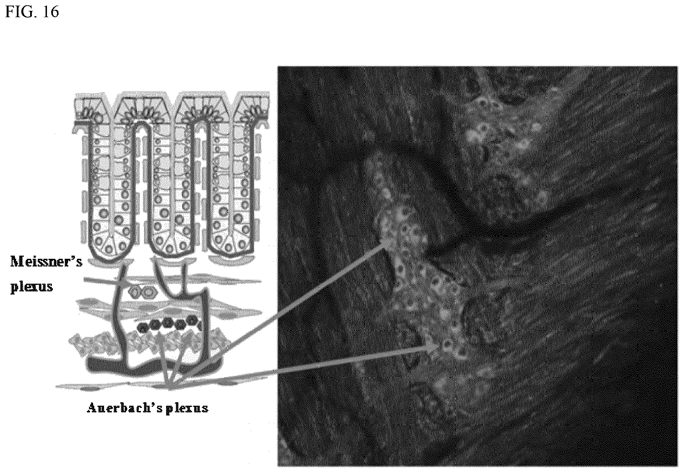

In an embodiment of the present invention, Auerbach's plexus in muscle layers can be visualized by a laser microscope after vital staining with curcumin. FIG. 16 is an example in which large intestine of a mouse was stained with curcumin from serosal side, and then Auerbach's plexus in a muscle layer was visualized by a multiphoton laser microscope. According to FIG. 16, the perikaryon can be identified by curcumin staining. Curcumin stains only the cytoplasm, but does not stain the nucleus in perikaryon, so that the cytoplasm is viewed as a positive image and the nucleus is viewed as a negative image. Accordingly, the morphology of perikaryon can be accurately determined. Cancer cells develop in the epithelial and glandular layers (1) (see FIG. 5a), and then expand and move to other layers (called cancer cell invasion). In that case, it is known that cancer cells tend to move along blood vessels and peripheral nerves. Therefore, the ability to visualize Auerbach's plexus in muscle layer by the vital staining of curcumin indicates that the invasion pathway of cancer can be visualized, which is useful for determining the extent of cancer invasion. In addition, early cancer and advanced cancer can be determined.

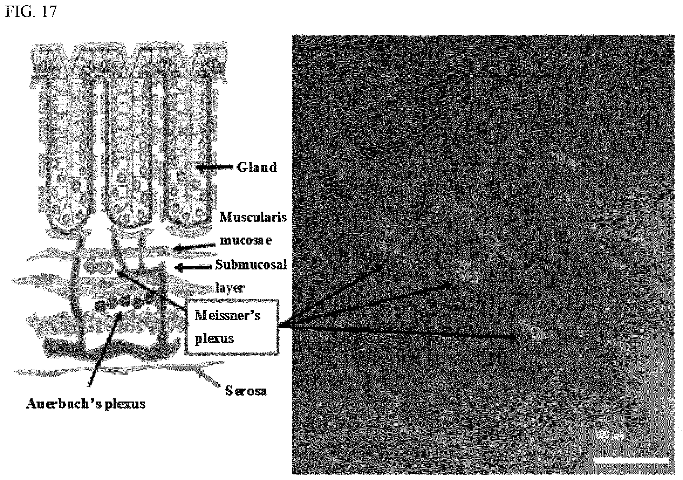

In an embodiment of the present invention, autonomic plexus in muscle layer (Meissner's plexus) can be visualized with a laser microscope after vital staining with curcumin. FIG. 17 shows an example in which Meissner's plexus was visualized with a laser microscope after staining large intestine of a mouse with curcumin from serosa side. According to FIG. 17, perikaryon can be identified by curcumin staining. The Meissner's plexus is located in submucosa, and one to several neuronal cells form a cluster. Curcumin stains only the cytoplasm, but does not stain the nucleus in perikaryon, so that the cytoplasm is viewed as a positive image and the nucleus is viewed as a negative image. Accordingly, the morphology of perikaryon can be accurately determined.

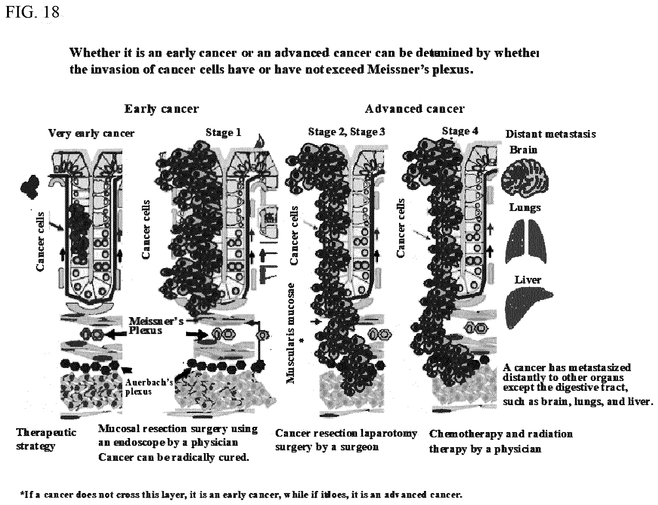

With reference to FIG. 18, a primary lesion necessarily remains inside mucosal epithelium, because cancer generally arises from mucosal epithelial cells. When the cancer tissue grows, cancer cells invade from inside the mucosal epithelium to deep parts. If the cancer cells have not yet invaded or reached the muscularis mucosae and Meissner's plexus, the cancer is determined as an early cancer. On the other hand, if the cancer cells have invaded or reached the muscularis mucosae and Meissner's plexus, the cancer is determined as advanced cancer.

As summarized in FIG. 39, if cancer cells have not yet invaded or reached the muscularis mucosae and Meissner's plexus, the cancer is determined as early cancer. On the other hand, if cancer cells have invaded or reached Meissner's plexus and the smooth muscle layer, the cancer is determined as advanced cancer. In addition, when cancer cells have invaded or reached Auerbach's plexus, the infiltration range of the advanced cancer is confirmed. As described above, by vital staining with curcumin and obtaining continuous tomographic images (representative 3 focal planes) from Auerbach's plexus to Meissner's plexus until the bottom of intestinal gland using a multiphoton laser microscope, it is possible to confirm the state and degree of cancer progression, and propose methods for treatment or surgery to a patient with cancer.

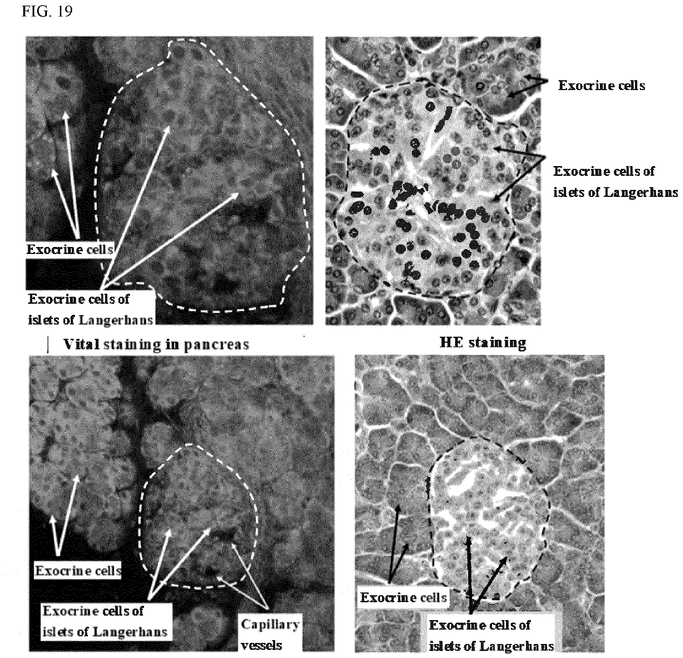

According to the present invention, various biological tissue can be visualized. FIG. 19 is a photograph in which exocrine cells of pancreas and islets of Langerhans are visualized with a laser microscope after vital staining by intraperitoneal administration of curcumin to a mouse. FIG. 19 further shows hematoxylin-eosin (HE) staining images of pancreatic tissue. As a result, exocrine cells and endocrine cells of islets of Langerhans (inside the dashed line) can be distinguished based on cell size and arrangement. It can be seen that endocrine cells are small, dozens of them aggregate in a globular shape, and their inside is rich in capillaries. Exocrine cells are larger than endocrine cells, and have a large number of secretory granules inside, which are clustered in groups of several cells. By intraperitoneal administration of a sterilized curcumin solution, the islets of Langerhans in pancreas can be visualized. Accordingly, it can be used for diagnosis of pancreatic cancer as well as diabetes and insulinoma in endocrine field.

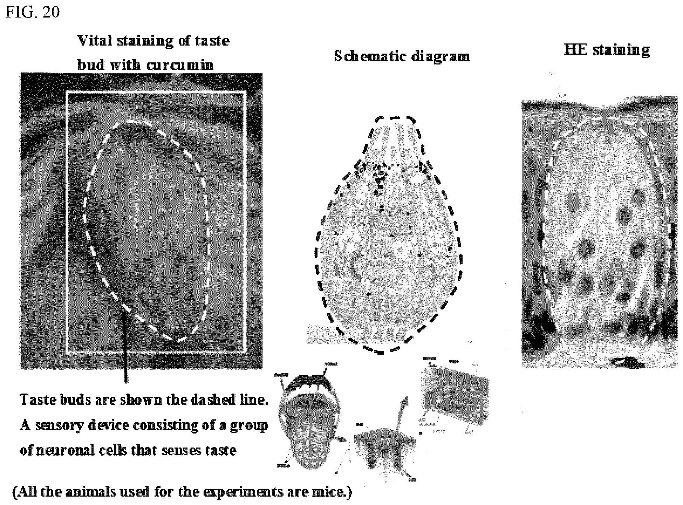

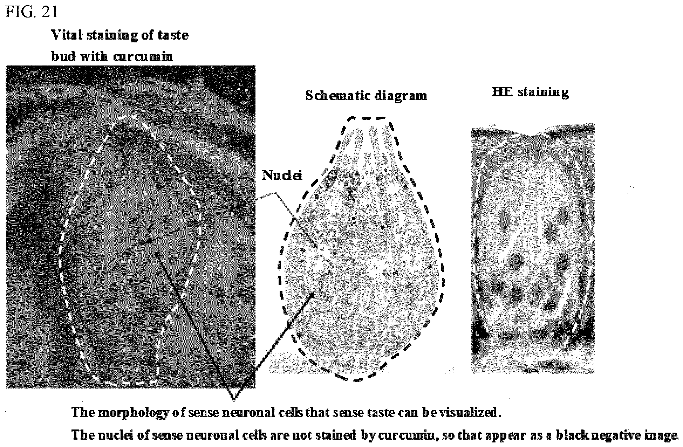

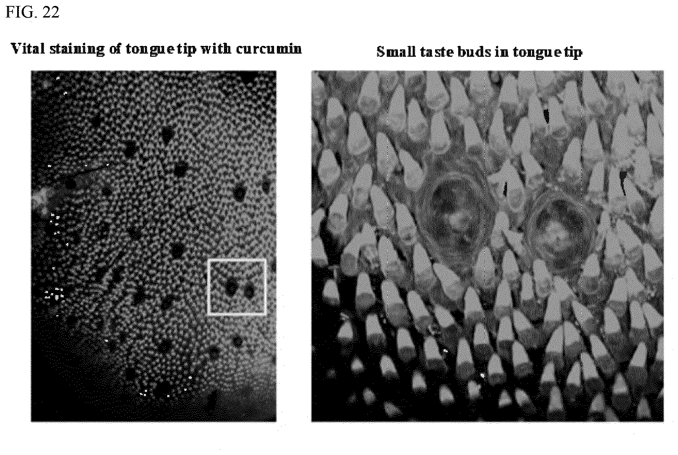

FIG. 20 to FIG. 22 are diagrams in which a taste bud, a taste sensory device, is visualized by a laser microscope after vital staining by coating curcumin on mouse tongue mucosa. FIG. 20 and FIG. 21 further show hematoxylin-eosin staining images of taste buds. The nuclei of sensory neuronal cells are not stained with curcumin, so that they are recognized as black negative images. By coating curcumin on oral mucosa, the neuronal cells of a taste bud, which is a taste sensory device, can be visualized. Accordingly, taste disorders can be examined in otolaryngologic field.

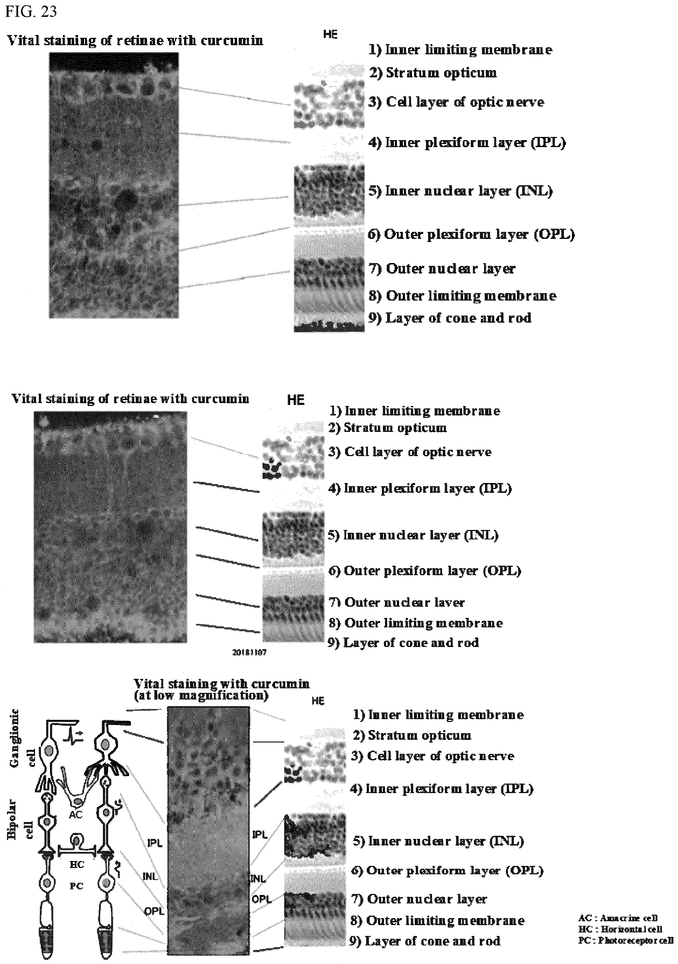

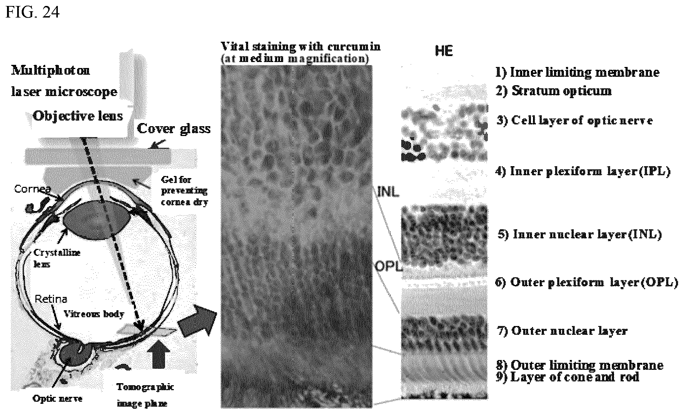

FIG. 23 and FIG. 24 are diagrams in which a retinal neuronal cell group was visualized with a multiphoton laser microscope after vital staining by intraperitoneal administration of curcumin to a mouse. These figures include hematoxylin-eosin staining images of retinal tissue. As indicated by the figures, retinal neuronal cell groups and synapse can be visualized. By oral administration or intravitreal injection of curcumin, the neuronal cell groups and synapses can also be visualized. In ophthalmic field, for example, stages of diabetic retinopathy, macular degeneration, retinal degeneration, proliferative vitreoretinopathy, glaucoma, or edema retinoblastoma can be determined.

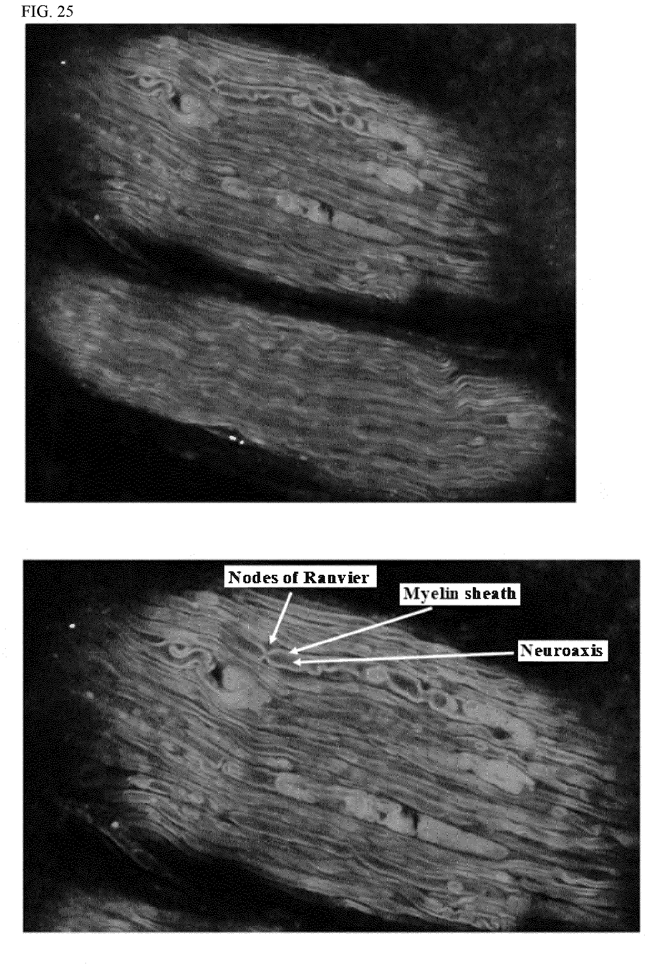

Furthermore, FIG.25 shows photographs of mouse peripheral nerve fibers (sciatic nerve fibers) vitally stained with curcumin and imaged with a multiphoton laser microscope, in which myelin sheath and nodes of Ranvier of myelinated nerves were visualized.

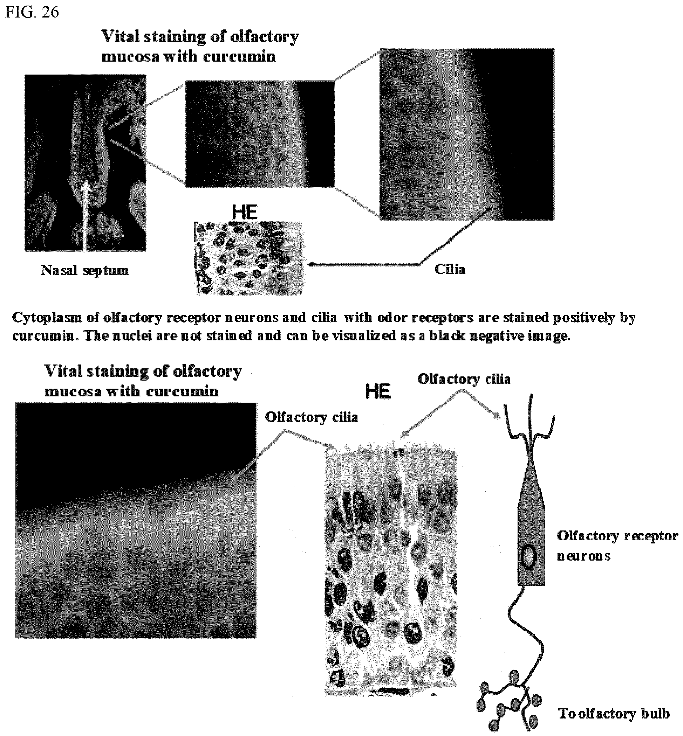

FIG.26 also shows photographs of olfactory receptor neurons that are odor sensory cells can be stained with curcumin and visualized with a laser microscope. For comparison, Hematoxylin-eosin staining images are also shown in FIG. 26. The cytoplasm of olfactory receptor neurons and cilia having odor receptors are stained positively with curcumin. Since a nucleus is not stained, it is viewed as a black negative image. By coating curcumin on nasal mucosa, olfactory receptor neuronal cells, which are odor sensing cells, can be visualized. Accordingly, olfactory disorders can be examined.

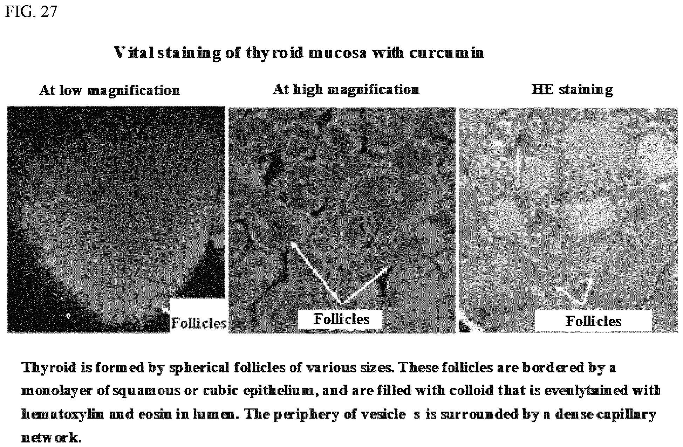

In an embodiment of the present invention, thyroid can be visualized with a laser microscope after vital staining with curcumin (FIG. 27). The structure of vesicles is stained by vital staining thyroid with curcumin. Thyroid is formed by spherical follicles of various sizes. These follicles are bordered by a monolayer of squamous or cubic epithelium, and are filled with colloid that is evenly stained with hematoxylin-eosin in lumen. It can be seen that the periphery of the vesicle is surrounded by a dense capillary network.

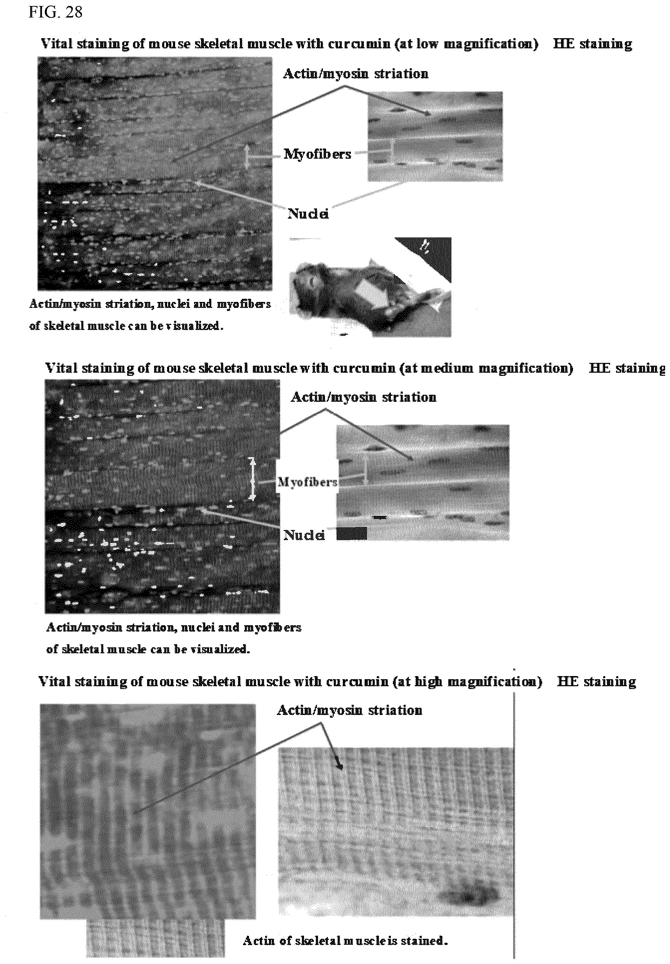

In an embodiment of the present invention, actin/myosin striation, nuclei, and myofibers of skeletal muscle can be visualized with a laser microscope after vital staining by coating curcumin onto fascia (FIG. 28). By coating a sterilized solution of curcumin on fascia, myofibers of skeletal muscle and actin molecules can be visualized. Accordingly, it can be possibly applied to morphological photo biopsy diagnosis of muscle weakness/frail syndrome.

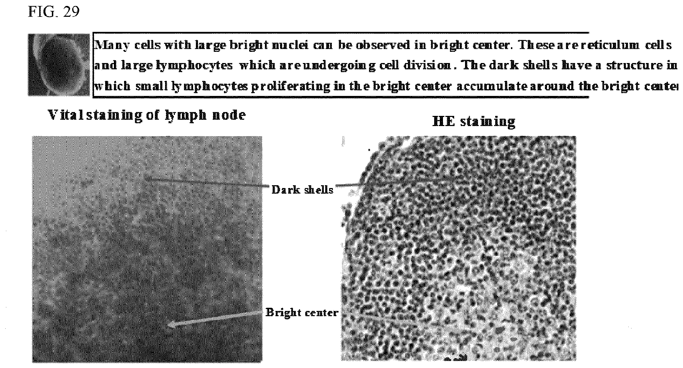

In an embodiment of the present invention, the structure of bright center and dark shell of the secondary nodules of lymph nodes can be visualized with a laser microscope after vital staining by coating curcumin onto lymph nodes (FIG. 29). In the bright center, many cells with large bright nuclei can be observed. These cells are reticulum cells and large lymphocytes which are undergoing cell division. The dark shells have a structure in which small lymphocytes proliferating in the bright center accumulate around the bright center. The cell structure inside lymph node can be visualized by coating a sterilized solution of curcumin to the surface. Accordingly, in urology field and gastrointestinal surgery field, it is possible to determine the presence or absence of lymph node metastasis of cancer during laparoscopic robotic surgery.

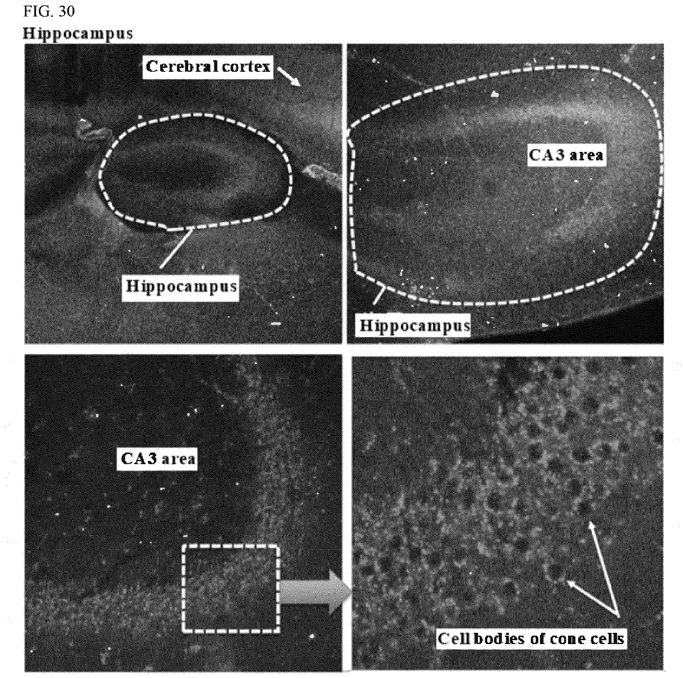

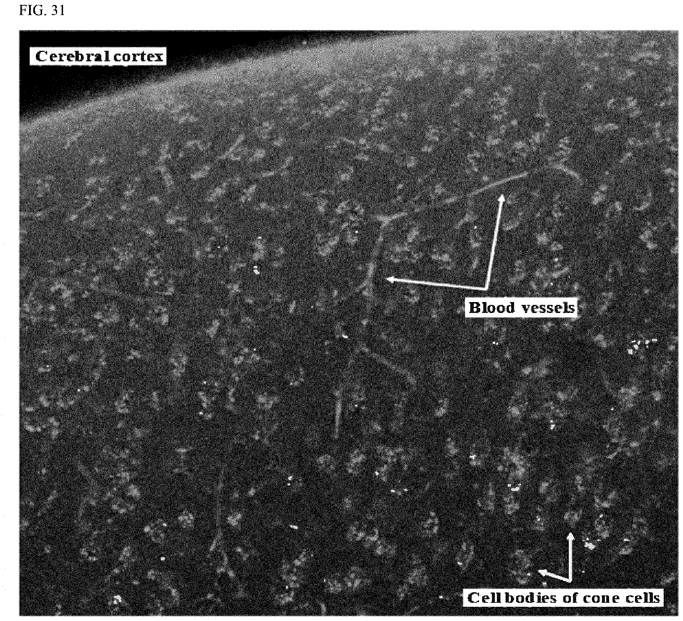

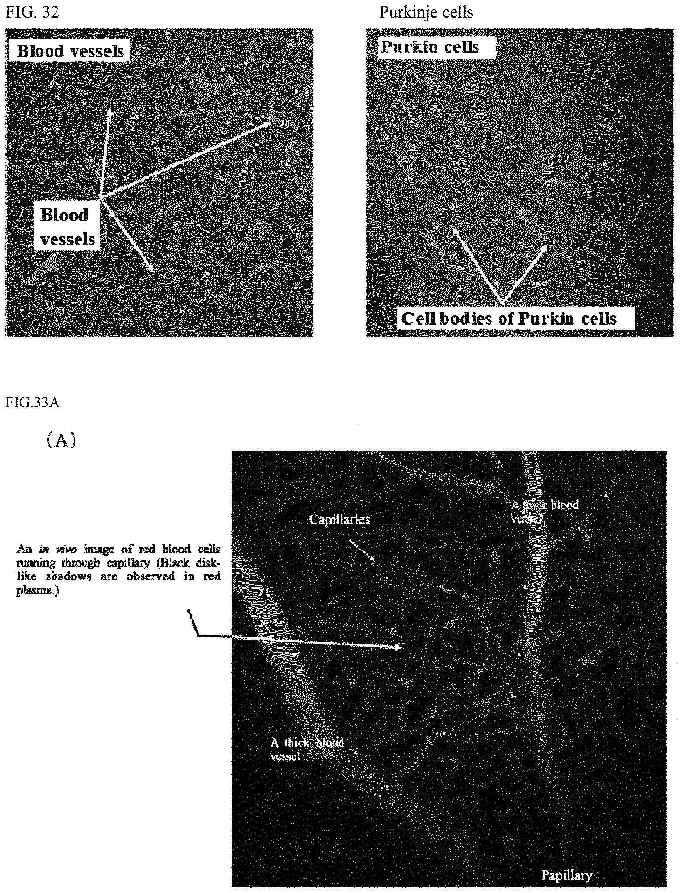

In an embodiment of the present invention, pyramidal cell bodies can be visualized in hippocampal CA3 area by laser microscopy after intraperitoneal administration of curcumin (FIG. 30). In cerebral cortex, cell bodies of blood vessels and cone cells can be visualized (FIG. 31). In cerebellum, cell bodies of blood vessels and Purkinje cells can be visualized (FIG. 32). By using the present invention, Alzheimer's disease, cerebral infarction, cerebral hemorrhage, subarachnoid hemorrhage, spinocerebellar degeneration, and the like, which are accompanied by degeneration of brain tissue, can be examined.

In an embodiment of the present invention, it is possible to visualize retinal blood vessels by a multiphoton laser microscope after intraperitoneal administration of sulforhodamine 101 (FIG. 33). In addition to the thick blood vessel of the retina, individual capillaries are clearly visualized in vivo (FIG. 33A), and the capillaries of the retina are shown to be anastomosed in a loop shape (FIG. 33B). Therefore, the lesion occurring in the retina can be grasped quickly and reliably. In addition, by visualizing the capillaries of the retina, the red blood cells passing through the lumen of the capillaries can also be visualized as black shadows (FIG. 33C). Since the visualized image by the laser microscope can be photographed in real time, the moving speed of the red blood cells in the capillary can be measured by obtaining a plurality of microscopic images by shifting the photographing time (FIG. 34). Furthermore, the number of red blood cells moving in the capillary within a certain period of time can also be measured (FIG. 33C). In the normal mouse, since the fluorescence staining concentration of the sulforhodamine 101 in the capillary vessel is almost uniform and the fluorescent image derived from the staining agent is not recognized outside the capillary vessel, leakage of the staining agent to the outside of the capillaries is not recognized (FIG. 35A). On the other hand, in the retinal vessel of the model mouse with diabetes, the tube diameter on the peripheral side of the capillary vessel is reduced according to the observation with a laser microscope after the administration of sulforhodamine 101. Moreover, the leakage of the staining agent from the blood vessel is recognized (FIG. 35B) according to the observation of fluorescent image wherein periphery of the capillary vessel appears to be covered by mist. The leakage of curcumin to the outside of capillaries observed in a model mouse with diabetes indicates the breakdown of blood-brain barrier in the capillary vessel of the retina. That is, according to the present invention, it is possible to identify an early change in diabetes by visualizing the retinal blood vessel. As described above, the present invention can provide important means in the determination of early lesions of ophthalmic diseases or ophthalmic symptoms, such as macular degeneration, retinal degeneration, diabetic retinopathy, retinoblastoma, proliferative vitreous retinopathy, glaucoma, retinal detachment, and retinal edema.

According to FIGS. 36A and 36B, the present invention makes it possible to coagulate blood vessels by laser one by one while visualizing retinal vessels under a laser microscope. That is, under a laser microscope, the target blood vessel can be selected by visualizing the blood vessel with a low-output laser, and the target blood vessel can be coagulated in real time by raising the laser output while confirming the target site. Therefore, the present invention is very useful for retinal therapy.

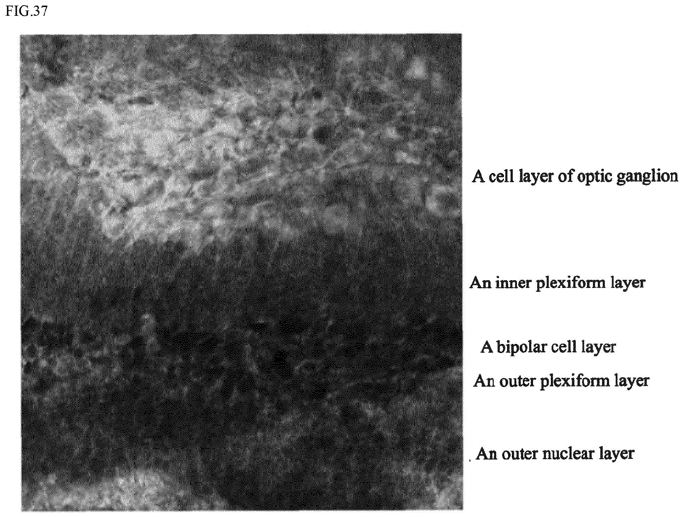

In an embodiment of the present invention, the nerve cell layer of retina can be visualized by vital staining with curcumin (FIG. 37). According to FIG. 37, it can be seen that in the layer structure of retina, the cell layer of optic ganglion, inner plexiform layer, bipolar cell layer, outer plexiform layer, and outer nuclear layer can be identified. It can be seen that the blood vessel travel and the optic nerve bundle in retina can be visualized at the same time by double staining with blood vessel staining dye (sulforhodamine 101) and curcumin (FIG. 38A). In addition, in curcumin vital staining, it is shown that the nucleus of optic nerve and small/large ganglion cells can be visualized as negative signal and the cytoplasm can be visualized as positive signal, so that they can be clearly identified. Accordingly, the present invention is very useful for detecting retinal diseases.

Diseases in which morphology, relative position and arrangement in tissue, and number of cells in tissue are changed or fluctuated in all organs compared with normal tissue can be detected or diagnosed according to the present invention. The diseases include those exemplified above, such as cancer, diabetes, diabetic retinopathy, macular degeneration, retinal degeneration, taste disorder, olfactory disorder, Alzheimer's disease, and cerebral infarction, etc. However, they are not limited thereto. On the other hand, diseases in which only the function of cells changes or fluctuates, for example, schizophrenia, cannot be detected or diagnosed according to the present invention.

The method of the present invention is characterized in that by performing tissue staining and laser irradiation from serosal side of an organ suspected of presence of cancer, cancer tissue can be visualized before surgical operations or before resection of an affected part during surgical operations. In an actual surgery, visualizing the location or invasion extent of cancer cells and marking the resection site of an organ, that is, the margin of the cancer tissue is greatly supportive for a surgeon. For this purpose, it is preferable to color the location or invasion extent of the cancer cells on serosa. For such coloring, a surgical thread or tape may be used as a well-known surgical biomarker, or a marking dye may be used. Examples of surgical biomarkers include sodium sulfobromophthalein, indocyanine green, sodium fluorolein, methylene blue, indigo carmine, toluidine blue, and picotanine blue, etc. To enhance tissue adhesion of these marking dyes, a thickener such as sodium carboxymethylcellulose, sodium hyaluronate, gum arabic, and the like can be mixed.

As a tip probe for laser irradiation, a stick type objective lens with a diameter of about 5 mm and a needle type objective lens with a diameter of about 0.3-2 mm can be used in addition to a normal objective lens with a diameter of about 25 mm.

During a surgical operation for cancer treatment, notifying operators that cancer cells have been detected is useful as a supportive method for assisting operators in successfully performing cancer treatment. Such notification to the operators can be made by sound or light. In particular, a system for notifying the presence of cancer tissue by comparing images photographed with a laser microscope with cancer tissue images stored in a database in advance is preferable as a means for preventing cancer tissue from being left behind.

The test conditions in the above tests, including the preparation of cell staining solutions, the animals used, the methods for preparing model mice with colon cancer, and the conditions of laser irradiation are as follows.

### [Preparation of cell staining solutions]

100 mg of curcumin (Tokyo Kasei, cat. # C2302, purity 97.0%) was suspended in 5 mL of ethanol, and further diluted 10-fold with ethanol. It was mixed with the same amount of glycerin, and further diluted 10-fold with glycerin. The mixture was mixed with the same amount of purified water to obtain a staining solution of curcumin. Regarding Red #106, its staining solution is obtained by dissolving the powder in saline to achieve a concentration of 1 mg/mL.

### [Animals]

C57BL/6N mice purchased from Japan SLC, Inc. were used in the tests. All tests were performed on male, 8-week-old mice weighing 20∼25 grams.

### [Preparation of model mice with colon cancer]

Model mice with colon cancer were prepared by intraperitoneally administering 10 mg/kg of azoxymethane (AOM) dissolved in saline 4 times at weekly intervals to the C57BL/6N mice.

### [Preparation of model mice with diabetes]

60 mg/kg of streptozotocin dissolved in physiological saline was intraperitoneally administered to the C57BL/6N mice. A week later, their blood sugar was measured and mice with blood sugar of 300 mg/dl or more were used as model mice with diabetes.

### [Conditions of laser irradiation]

A multiphoton laser microscope of FVMPE-RS (Olympus) was used with the irradiation wavelength of 800 nm, wherein the laser irradiation was performed at 5.8∼48.2% of its full power output. A confocal laser microscope of FV1000 (Olympus) was used with the irradiation wavelength of 488 nm and 594 nm, wherein the laser irradiation at 488 nm was performed at 15∼29.4% of its full power output, and the laser irradiation at 594 nm was performed at 13∼13.5% of its full power output. The direction of irradiation and staining from serosal or luminal side are indicated in the figures.

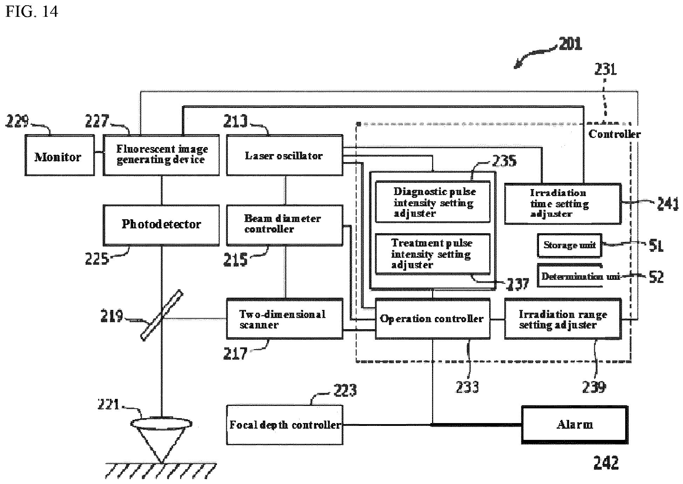

In an embodiment of the present invention, as shown in FIG. 14, the cancer testing device (201) is equipped with laser oscillator (213), beam diameter controller (215), two-dimensional scanner (217), dichroic mirror (219), objective lens (221), focal depth controller (223), photodetector (225), fluorescence image generating device (227), monitor (229) and controller (231).

As the laser oscillator (213), one capable of adjusting the output of pulsed laser beam with a pulse width in the extent of tens to hundreds of femtoseconds and a pulse repetition frequency in the extent of tens to hundreds of MHz is used.

The beam diameter controller (215) is a beam expander that changes the beam diameter of pulse laser beam according to a beam diameter adjustment signal from the controller (231).

The two-dimensional scanner (217) comprises, for example, two Galvano mirrors, and changes the focal position of pulsed laser beam in two axial directions perpendicular to optical axis.

The dichroic mirror (219) separates the fluorescence generated in a cancer-related gene product of living cells by irradiating with pulsed laser beam.

The objective lens (221) condenses the pulsed laser beam emitted from the laser oscillator (213) on living cells, while condensing the fluorescence generated in a cancer-related gene product according to multiphoton absorption phenomenon. The objective lens (221) is movable in optical axis direction by a focal depth controller (223) based on a control signal, and can adjust the focal position.

The photodetector (225) detects the fluorescence generated in a cancer-related gene product and converts it into electric signals corresponding to fluorescence intensity.

The scanning state of the two-dimensional scanner (217) and the adjustment position (position in the depth direction) of the focal depth controller (223) are parameters representing coordinates of focal position. The fluorescence image generating device (227) stores the parameters representing these coordinates and the electric signal (that is, the fluorescence intensity) transmitted from the photodetector (225) in association with each other, processes these data, and generates fluorescence images. A generated fluorescent image is displayed on the monitor (229).

The controller (231) comprises operation controller (233), diagnostic pulse intensity setting adjuster (235), irradiation extent setting adjuster (239) and irradiation time setting adjuster (241). The operation controller (233) controls the operations of the laser oscillator (213), the beam diameter controller (215), the two-dimensional scanner (217) and the focal depth controller (223).

In order to perform a test, a pulse laser beam intensity is set by the diagnostic pulse intensity setting adjuster (235) at an intensity suitable for achieving a fluorescent image of cancer-related gene expression pattern.

The irradiation extent setting adjuster (239) sets an extent in which living cells are irradiated with pulsed laser beam. The operation controller (233) controls the operations of the two-dimensional scanner (217) and the focal depth controller (223), thereby irradiating pulsed laser beam at the set irradiation extent and depth and condensing it. The irradiation time setting adjuster (241) sets the time for irradiating pulse laser beam on living cells. Then, the operation controller (233) controls the output of the laser oscillator (213) so that pulse laser beam is emitted for a set time.

In an embodiment, the controller (231) has a storage unit (51) and a determination unit (52). That is, the cancer testing device (201) determines the malignancy and prognosis of malignant transformation of a living cell group in real time based on the staining state of the living cell group in the images achieved by photographing.

By using the cancer testing device (201), the malignancy of malignant transformation is determined based on staining state of cancer-related gene expression pattern of living cell groups, so that the malignant transformation of the living cell groups can be grasped at an early stage. Further, since the malignancy of malignant transformation can be grasped by the expression state of cancer-related genes, the prognosis of cancer patients can be known.

The cancer testing device (201) is equipped with a treatment pulse intensity setting adjuster (237), so that a pulse laser beam intensity that is high enough to destroy living cells for performing a treatment can be set. Accordingly, early cancer treatment can be performed on the cancer cell group discovered.

In addition, the cancer testing device (201) can be used in various forms.

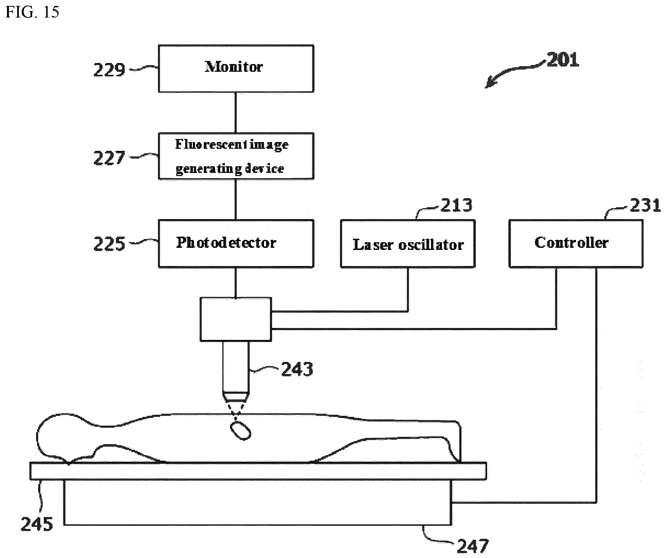

For example, as shown in FIG.15, to perform a cancer test, beam diameter controller (215), two-dimensional scanner (217), an optical system consisting of a dichroic mirror (219), objective lens (221) and an optical path therebetween, and focal depth controller (223) are provided in the laser irradiation head (243), and a patient fixing table (245) for mounting a patient together with a moving device (247) are further provided.

Besides, for example, the malignancy of malignant transformation may also be determined from images taken with the cancer testing device (201) when the shaved living cell group is placed in a tray (sample stage) after being scrapped off a part of a living cell group from a patient. In this case, coating the staining agent (45) to a living cell group may be performed before the living cell group is scraped, or may be performed after the living cell group is scraped but before photographing. In addition, the cancer testing device can also be used to accurately cut out the affected area of cancer in real time during a surgery, or to show that it was cut out accurately after resection. When used in a surgery, an accurate position in cm units is known in advance by a normal endoscope, CT, X-ray imaging, or the like in order to specify a site on which the surgery is to be performed. By using the cancer testing device of the present invention during a surgery, the boundary between cancer tissue and normal tissue can be accurately grasped. It is possible to remove cancer radically while minimizing the extent of tissue removal, which significantly reduces the burden on patients undergoing cancer removal surgery.

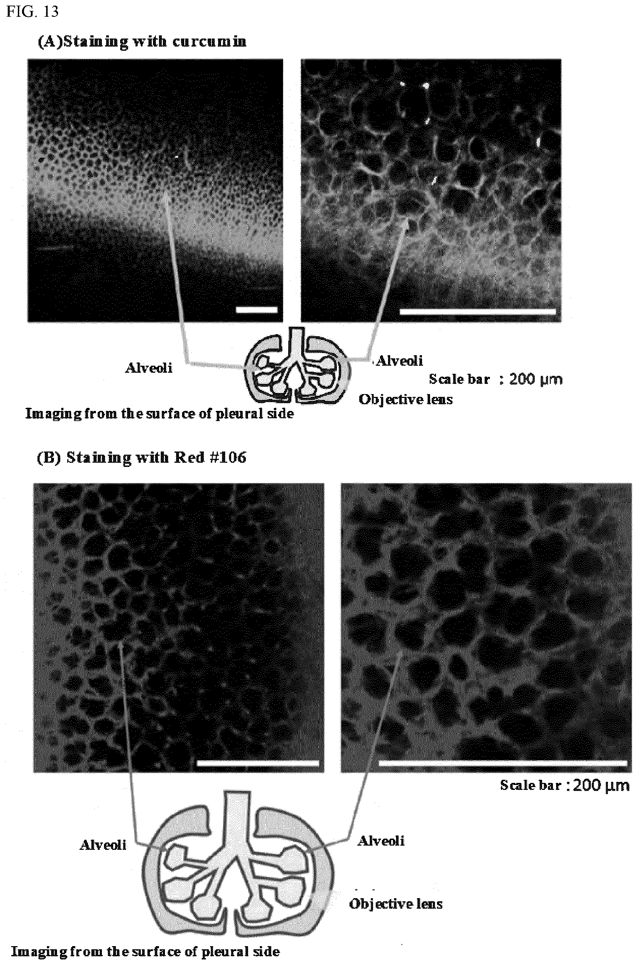

Specifically, as described with reference to the schematic diagrams and images in FIGS. 8 to FIGS. 13, after staining in advance with curcumin or the like, the confirmation can be made on the monitor (229). It can also be notified by sound such as a buzzer or by light such as a flash or a color light from an alarm. The effect in this case is that the boundary between normal cells and early cancer parts can be clearly determined from images from the center of advanced cancer. It can be determined instantaneously whether it is an early cancer cell, a normal cell, a neuronal cell, a blood vessel, or a noise from the fluorescence intensity, fluorescence color and shape of cells (nucleus, crypt, etc.). Accordingly, as described above, marking the margins of cancer tissue is greatly supportive to a surgeon. In this case, a surgical thread or tape may be used as the biometric marking for surgery, or a marking dye may be used. However, it is particularly useful to provide a nozzle for marking dye in conjunction with the objective lens (221) in FIG. 14. In addition, it is also effective to increase laser irradiation intensity to partially burn peripheral contour of cancer tissue or to transpire it into a shape shown in the dashed line.

In determining the peripheral portion, the movable portion including the objective lens of the cancer testing device (201) is moved in the X-Y direction by a distance including the peripheral portion and center of cancer, and the point where the fluorescence density mostly decreases is marked. Thereafter, by rotating the movable portion at an angle of, for example, about 5 degrees and repeating the same moving sweep, the outermost peripheral edge portion to be resected including the advanced cancer can be marked.

As described above, the present invention provides a method for identifying tissue and cells necessary for a surgeon to immediately make pathological diagnosis during a surgery, thereby enabling radical resection of cancer while reducing the extent of tissue removal. Consequently, the burden on patients undergoing cancer removal surgeries is greatly reduced.

### STATEMENT REGARDING THE RESULTS OF COMMISSIONED RESEARCHES BY THE GOVERNMENT

This invention is achieved as the results of commissioned researches by Japan Agency for Medical Research and Development as National Research and Development Agency for the year of 2016 under the project entitled as "Strategic promotion program for translational research Seeds B", "Development of rapid imaging pathological diagnosis technology using vital staining and laser microscopic endoscopy that enables real-time optical biopsies of lesions in biological tissue", and 2018-2019 under the project entitled as "Strategic promotion program for translational research Seeds B", "Development of rapid imaging pathological diagnosis technology using vital staining and confocal laser microscopic endoscope that enables real-time optical biopsies of lesions in biological tissue", "Project of elucidation and control of aging mechanism", and "Support for microstructure analysis with electron microscopes". Accordingly, the Article 17 of Industrial Technology Enhancement Law is applied to this application.

## Claims

1. A method for detecting lesion **characterized by** administering to an organ a cell staining agent that enables observation of biological tissue with laser irradiation, then irradiating the organ with multiphoton laser or confocal laser, obtaining histological images inside of a lesion in the organ, and conforming the interface between the normal site and the lesion site.

2. The method according to claim 1, wherein the inside of a lesion is a micro lesion with a diameter of about 5∼500 µm.

3. The method according to claim 1 or 2, wherein the organ is irradiated from its serosal side or lumen with multiphoton laser or confocal laser.

4. The method according to any one of claims 1∼3, wherein the cell staining agent is one or more staining agents selected from the group consisting of sulfuretin, curcumin, Red #3 (erythrosine), and Red #106.

5. The method according to any one of claims 1∼4, wherein the administration of a cell staining agent is performed by coating, dropping or spraying from the serosal side of an organ.

6. The method according to any one of claims 1∼4, wherein the administration of a cell staining agent is performed by coating, dropping or spraying from the lumen of an organ.

7. The method according to any one of claims 1∼4, wherein the administration of a cell staining agent is oral administration, intravenous administration, intraperitoneal administration, subcutaneous injection, intramuscular injection, intraorgan injection, intrathoracic administration, or subarachnoid administration.

8. The method according to any one of claims 1∼7, wherein the laser irradiation is performed by using a multiphoton laser microscopic endoscope, a confocal laser microscopic endoscope, or a laser fluorescent microscopic endoscope.

9. The method for detecting cancer cells, **characterized by** using the method according to claim 8 to visualize cancer cells.

10. The method according to claim 9 for determining invasion of cancer to regional lymph node tissues when cancer cells exist in an organ suspected of presence of cancer, which comprises administering to lymph node tissues a cell staining reagent that enables observation of biological tissue with laser irradiation by dropping from the surface covering the lymph node tissues or injecting into the lymph node, and then irradiating the lymph node tissues with multiphoton laser or confocal laser.

11. The method according to claim 9, **characterized by** staining tissue in an organ suspected of presence of cancer cells with curcumin or sulfuretin, then laser irradiating the organ tissue from its serosal side or lumen using a multiphoton laser microscopic endoscope, a confocal laser microscopic endoscope, or a laser fluorescent microscopic endoscope, and identifying normal or cancer cells based on visualized images obtained on cytoplasmic and nuclear morphology of the cells present in the organ tissue.

12. The method according to claim 11, wherein the organ is a respiratory, digestive, or genitourinary organ.

13. The method according to claim 9, **characterized by** staining tissue in an organ suspected of presence of cancer with curcumin, then laser irradiating the organ tissue from its serosal side or a lumen using a multiphoton laser microscopic endoscope, a confocal laser microscopic endoscope, or a laser fluorescence microscopic endoscope, comparing the crypt structures of cancer tissue and normal tissue present in the visualized organ tissue, and determining the lesion site as cancer according to the observation of disappearance of the regular crypt structure found in normal tissue and populations of disordered cell proliferation of cancer cells that do not have the crypt structure.

14. The method according to claim 9, **characterized by** staining tissue in an organ suspected of presence of cancer with Red #106, then laser irradiating the organ tissue from its serosal side or lumen using a multiphoton laser microscopic endoscope, a confocal laser microscopic endoscope, or a laser fluorescent microscopic endoscope, comparing the patterns of the capillaries around cancer cells and normal cells in the visualized organ tissue, and detecting the cancer cells according to the observation of disappearance and/or deformation of the capillaries in the regular crypt structure found in normal tissue.

15. The method according to claim 9, **characterized by** vital staining epithelial cells and cancer cells with curcumin, or connective tissue and capillaries with Red #106 in an organ suspected of presence of cancer, then laser irradiating the organ tissue from its serosal side or lumen using a multiphoton laser microscopic endoscope, a confocal laser microscopic endoscope, or a laser fluorescent microscopic endoscope, conforming the boundary between the cancer cells and the connective tissue existing in the visualized organ tissue, and determining the infiltration of the cancer cells.

16. The method according to claim 9, which comprises staining an organ tissue with curcumin or sulfuretin in an organ suspected of presence of cancer, then laser irradiating the organ tissue from its serosal side or lumen using a multiphoton laser microscopic endoscope, a confocal laser microscopic endoscope or a laser fluorescent microscopic endoscope, and visualizing Meissner's plexus or Auerbach's plexus present in the organ tissue.

17. The method according to claim 16, **characterized in that** when a primary lesion of cancer is in mucosal epithelium, if the cancer cells have invaded or reached Meissner's plexus, the cancer is determined as an advanced cancer.

18. The method according to claim 16, **characterized in that** when a primary lesion of cancer is in mucosal epithelium, if the cancer cells have invaded or reached the Meissner's plexus and smooth muscle layer, the cancer is determined as an advanced cancer.

19. The method according to claim 16, **characterized in that** when a primary lesion of cancer is in mucosal epithelium, if the cancer cells have not yet invaded or reached Meissner's plexus, the cancer is determined as an early cancer.

20. The method according to claim 16, **characterized by** comprehensively observing the interface between cancer tissue and normal tissue surrounding the ultra-early cancer tissue, and determining whether or not the cancer has infiltrated and metastasized according to the image of the interface.

21. The method according to any one of claims 9∼20, which further comprises notifying the detection of cancer cells by sound or light.

22. The method for treating cancer patients by removing cancer cells one by one from serosal side or lumen, **characterized by** using any one of the methods according to claims 9∼21.

23. The method for confirming cancer cells remaining *in vivo* from serosal side or lumen after a surgery, and removing the cancer cells one by one, **characterized by** using any one of the methods according to claims 9∼21.

24. A method for diagnosing the tissue-type of lung cancer, **characterized by** using any one of the methods according to claims 9∼21.

25. A method for treating patients with lung cancer, **characterized by** using the method according to claim 24.

26. The method according to claim 25, wherein lung cancer cells are destroyed by laser transpiration with laser beam.

27. A method for visualizing brain tissue, **characterized by** using the method according to claim 8 to fluorescently label neurons in the brain tissue with a staining agent and determine the morphology of the neurons.

28. A method according to claim 27, wherein the brain tissue is cerebral cortex, hippocampus, amygdala, hypothalamus, or cerebellum.

29. A method for detecting a brain disease or brain symptom using visualized images obtained by the method according to claim 27 or 28.

30. A method according to claim 29, wherein the brain diseases or brain symptom comprise Alzheimer's disease, cerebral infarction, cerebral hemorrhage, subarachnoid hemorrhage, multiple sclerosis, and spinocerebellar degeneration.

31. A method for visualizing ocular tissue, **characterized by** using the method according to claim 8 to fluorescently label neurons in the ocular tissue with a staining agent and determine the morphology of the neurons.

32. A method according to claim 31, wherein the ocular tissue is retina.

33. A method for detecting an ophthalmic disease or ophthalmic symptom, using the visualized images obtained by the method according to claim 31 or 32.

34. A method according to claim 33, wherein the ophthalmic disease or ophthalmic symptom comprise macular degeneration, retinal degeneration, diabetic retinopathy, retinoblastoma, proliferative vitreoretinopathy, glaucoma, retinal detachment, and retinal edema.

35. A method for detecting whether or not cancer cells exist in lymph nodes during a laparoscopic surgery before lymph node resection, **characterized by** using the method according to claim 10.

36. A cancer immunotherapy **characterized by** visualizing the cancer cells that have metastasized to lymph nodes by the method according to claim 9, destroying only cancer cells one by one by laser transpiration, letting lymphocytes recognize the cancer-related antigens of the destroyed cancer cells, and letting activated lymphocytes attack cancer cells in the cancer primary lesion.

37. A method for diagnosing a disease that causes abnormality in location, number, shape, size, and arrangement of cells by visualizing cell structure of neuronal cells in digestive tract, brain and retina, sensory cells of taste and smell, endocrine cells, lymph nodes, skeletal muscle, lungs, pancreas, or liver by oral or intraperitoneal administration of curcumin, and imaging the visualized cell structure with a multiphoton laser microscopic endoscope, a confocal laser microscopic endoscope or a fluorescent microscope.

38. A method for destroying and removing abnormal cells one by one by laser irradiation in a disease diagnosed by using the method according to claim 37.

39. A cell removal method wherein when transplanted iPS cells are transformed into undifferentiated cells or cancer cells, these altered cells are visualized by the method according to claim 9, and only the altered cells are destroyed by laser ablation.

40. A method for diagnosing the cause of tonsillitis, **characterized by** using the methods according to claim 8.

41. A method according to claim 40, **characterized by** staining out polynuclear leukocytes, lymphocytes, and eosinophils by multiple staining with three or more kinds of edible dyes.

42. A method according to claim 41 regarding cell types of leukocytes infiltrating tonsils, which comprises steps of detecting as bacterial infectious tonsillitis when a large number of neutrophils infiltrate, detecting as allergic tonsillitis when a large number of eosinophils infiltrate, and detecting as viral infectious tonsillitis when a large number of lymphocytes infiltrate.

43. A method for visualizing skeletal muscle, **characterized by** using the method according to claim 8.

44. A method of analyzing the morphology of skeletal muscle by using the visualization method according to claim 43 to determine the cause for muscle weakness due to aging.

45. A method for analyzing the morphology of skeletal muscle by using the visualization method according to claim 43 to diagnose the lesions of sarcopenia and/or myasthenia gravis.

46. A method for detecting a lesion, comprising the steps of administering a staining agent to an organ for a sufficient period and at a sufficient amount that enable the visualization of the lesion in the organ, irradiating the organ with multiphoton laser or confocal laser, and visualizing the lesion of the organ.

47. Use of a cell staining agent for a lesion in an organ, **characterized by** administering the cell staining agent to the organ, then irradiating the organ with multiphoton laser or confocal laser, obtaining histological images inside of lesion in the organ, and determining the interface between normal site and lesion site.

48. A composition containing a cell staining agent for detecting a lesion in an organ, **characterized in that** after the composition is administered to the organ, the organ is irradiated with multiphoton laser or confocal laser, the histological cellular structure inside of lesion in the organ is imaged, and the interface between normal site and lesion site is determined.

49. Use of a multiphoton laser microscopic endoscope, a confocal laser microscopic endoscope, or a laser fluorescent microscopic endoscope for a lesion in an organ, **characterized by** administering a cell staining agent to the organ, then irradiating the organ with multiphoton laser or confocal laser, obtaining histological images inside of lesion in the organ, and determining the interface between normal site and lesion site.
